# EUROPÄISCHE PATENTSCHRIFT

(11) **EP 1 348 019 B1**
(45) Veröffentlichungstag und Bekanntmachung des Hinweises auf die Patenterteilung: **28.07.2010**
(21) Anmeldenummer: 01272041.3
(22) Anmeldetag: 27.12.2001
(51) Int. Cl.: C12N 5/0735, C12N 5/10, C12N 15/85

(54) **SYSTEM ZUR ZELL-UND ENTWICKLUNGSSPEZIFISCHEN SELEKTION DIFFERENZIERENDER EMBRYONALER STAMMZELLEN; ADULTER STAMMZELLEN UND EMBRYONALER KEINBAHNZELLEN**
SYSTEM FOR THE CELL-SPECIFIC AND DEVELOPMENT-SPECIFIC SELECTION OF DIFFERENTIATING EMBRYONIC STEM CELLS, ADULT STEM CELLS AND EMBRYONIC GERMLINE CELLS
SYSTEME DE SELECTION SPECIFIQUE AUX CELLULES ET AU DEVELOPPEMENT DE CELLULES SOUCHES EMBRYONNAIRES, DE CELLULES SOUCHES ADULTES, ET DE CELLULES GERMINATIVES EMBRYONNAIRES DE DIFFERENTIATION

(30) Priorität: 27.12.2000 DE 10065352; 27.07.2001 DE 10136702
(43) Veröffentlichungstag der Anmeldung: 01.10.2003
(60) Teilanmeldung: 10005766.0
(73) Patentinhaber: AXIOGENESIS AG, 50829 Köln (DE)
(72) Erfinder: FLEISCHMANN, Bernd, 50937 Köln (DE); BOHLEN, Heribert, 50733 Köln (DE); HESCHELER, Jürgen, 50968 Köln (DE); KOLOSSOV, Eugen, 50672 Köln (DE)
(74) Vertreter: Müller Fottner Steinecke
(86) Internationale Anmeldenummer: PCT/EP2001/015337
(87) Internationale Veröffentlichungsnummer: WO 2002/051987

(56) Entgegenhaltungen:
- DE-A- 19 727 962
- ANDRESSEN CHRISTIAN ET AL: "Nestin-specific green fluorescent protein expression in embryonic stem cell-derived neural precursor cells used for transplantation." STEM CELLS (MIAMISBURG), Bd. 19, Nr. 5, 2001, Seiten 419-424, XP002197255 ISSN: 1066-5099
- KLUG MICHAEL G ET AL: "Genetically selected cardiomyocytes from differentiating embryonic stem cells form stable intracardiac grafts" JOURNAL OF CLINICAL INVESTIGATION, NEW YORK, NY, US, Bd. 98, Nr. 1, 1996, Seiten 216-224, XP002142364 ISSN: 0021-9738 in der Anmeldung erwähnt
- MUELLER M ET AL: "Selection of ventricular-like cardiomyocytes from ES cells in vitro." FASEB JOURNAL, Bd. 14, Nr. 15, Dezember 2000 (2000-12), Seiten 2540-2548, XP002197256 ISSN: 0892-6638 in der Anmeldung erwähnt
- HESCHELER J ET AL: "Embryonic stem cells: A model to study structural and functional properties in cardiomyogenesis." CARDIOVASCULAR RESEARCH, Bd. 36, Nr. 2, November 1997 (1997-11), Seiten 149-162, XP002197257 ISSN: 0008-6363
- THOMSON J.A. ET AL: 'Embryonic stem cell lines derived from human blastocysts.' SCIENCE Bd. 282, 06 November 1998, Seiten 1145 - 1147
- GUHR A. ET AL: 'Current state of human embyronic stem cell research. An overview of cell lines and their use in experimental work' STEM CELLS Bd. 24, 2006, Seiten 2187 - 2191
- LAKSHMIPATHY U. ET AL: 'Efficient transfection of embryonic and adult stem cells.' STEM CELLS Bd. 22, Nr. 4, 2004, Seiten 531 - 543

## Beschreibung

Die Erfindung betrifft ein Verfahren zur Gewinnung von Herzzellen oder von sich zu Herzzelen differenzierenden Stammzellen, außer menschlichen embryonalen Stamm- und Keimbahnzellen.

Die in vitro Kardiomyogenese in der Kultur differenzierender embryonaler Stammzellen (ES) wurde als unbegrenzte Quelle von Herzmuskelzellen zur Transplantation in der Ersatztherapie des irreversibel geschädigten Herz-Gewebes vorgeschlagen (Klug et al., 1996). Eines der wesentlichem Hindernisse zur praktischen Ausführung dieses Ansatzes ist die relativ geringe Ausbeute an differenzierten, von ES abstammenden Herzmuskelzellen, die normalerweise nicht mehr als 1-3% einer differenzierenden ES-Zell-Gesamtpopulation ausmachen (Muller M. et al., 2000).

Darüber hinaus stellen die noch existierenden nicht differenzierten ES-Donorzellen in späteren Stadien der Differenzierung für den Empfänger eine potentielle Bedrohung bezüglich der Entwicklung von Tumoren dar. Deswegen wird die Aufgabe der Entwicklung eines wirksamen und hochspezifischen Selektionsverfahrens als einer der Ecksteine in der Zell-Therapie von Herzerkrankungen betrachtet.

Es wurde bereits früher dargestellt, daß eine mit Herzmuskelzellen angereicherte Population erfolgreich aus genetisch modifizierten ES-Zellen selektiert werden kann, die stabil mit einem Transgen eines α-Herz-Myosin-schwere-Kette-Promotor gesteuerten Arzneistoff-Resistenzgens der Aminoglykosid-Phosphotransferase (α-MHC-Neo) transfiziert wurden (Klug et al., 1996). Diese Arbeit zeigte weiterhin potentielle Probleme für die Entwicklung dieses Ansatzes für ein wirksames Verfahren in großem Maßstab:
a) Die Behandlung durch einen selektiven Arzneistoff (G418) wurde bezüglich einer adhärenten Kultur differenzierender ES-Zellen durchgeführt, wohingegen, unter dem Gesichtspunkt der Effektivität ebenso wie der technologischen Durchführbarkeit, der optimale Ansatz eine Aufbringung des selektiven Arzneistoffs direkt auf die Suspension der Aggregate von ES-Zellen - embryoiden Körpern (embryoid bodies = EBs) (Wobus et al., 1991) sein würde.
b) Die weiteren Experimente bezüglich des Einfügens genetisch selektierter Zellen in die Herzen von Empfängertieren werden durch die Arbeit signifikant kompliziert, das Schicksal der Einfügungen bei Fehlen spezifischer Lebens-Marker für Donorzellen nachzuweisen.

Die DE - A - 19727962 beschreibt embryonale Stammzellen nicht-menschlicher Säuger, die mit einem DNA-Konstrukt stabil transfiziert sind, welches eine DNA-Sequenz aufweist, die für ein nicht-zellschädigendes fluoreszierendes Protein kodiert, wobei diese DNA-Sequenz unter Kontrolle eines zell- und/oder entwicklungsabhängigen Promotors steht (Kolossov et al., 1998). Derartige rekombinante ES-Zellen weisen die nachfolgenden Nachteile auf:
1. Zwar können mit dieser Methode spezifische Zelltypen in vivo dargestellt werden, trotzdem gestaltet sich die Aufreinigung dieser vital gefärbten Zellen als sehr schwierig. Dies ist einerseits damit zu erklären, daß die Zellen von Interesse (z.B. Kardiomyozyten) nur ca. 1-3% der in EBs generierten Zellen ausmachen. Andererseits sind Zellaufreinigungsverfahren (z.B. fluorescence activated cell sorting, FACS) ideal für immunologische Zellen geeignet. Bei der Aufreinigung z.B. von Kardiomyozyten gehen jedoch viele Zellen zugrunde bzw. werden irreversibel geschädigt.
2. Ferner zeigt sich mit der Hygromycin-Aufreinigungsmethode an plattierten EBs, daß die nicht Hygromycin resistenten Zellen selbst nach 7-14tägigen Selektion nur schwer entfernt werden können. So kommt es nach der Transplantation trotz des zuvor erfolgten Einsatzes von Hygromycin als Selektionsmarker ebenfalls zur Tumorgeneration. Ähnliches gilt auch für eine Selektion mit Neomycin.

Es ist eine Aufgabe der vorliegenden Erfindung, ein neues System zur Selektion bzw. Auswahl von Zellen aus einer differenzierenden Kultur embryonaler Stammzellen, embryonaler Keimbahnzellen und adulter Stammzellen bereitzustellen, das die vorstehend beschriebenen Probleme vermeidet. Unter "System" ist eine Kombination von Selektionsverfahren, Zellen und Verwendung dieser Zellen und Verfahren insbesondere im medizinischen Bereich zu verstehen, wie sie in der vorliegenden Anmeldung beschrieben wird. Diese Aufgabe wird durch das Verfahren nach Anspruch 1 gelöst. Bevorzugte Ausführungsformen der Erfindung sind in den dem Anspruch 1 nachfolgenden Patentansprüchen beschrieben.

Offenbart wird ein System für eine herzell- und/oder entwicklungsspezifische Selektion differenzierender embryonaler Stammzellen, embryonale Keimbahnzellen und adulter Stammzellen durch kombinierte Anwendung von (Arzneistoff-) Resistenz- und detekierbaren Reportergenen unter gemeinsamer Kontrolle eines herzell- und/oder entwicklungsspezifischen Promotors. Vom Schutzbereich werden jedoch menschliche embryonale Stamm- und Keimbahnzellen, sowie deren Verwendung im erfinderischen Verfahren, ausdrücklich ausgeschlossen.

Die vorliegende Erfindung wird zunächst allgemein und anschließend an Hand von Beispielen auf Grundlage der genetischen Selektion von Herzzellen aus einer differenzierenden Kultur embryonaler Stammzellen beschrieben, die mit zwei Arten von Vektoren stabil transfiziert werden. Es wird betont, daß die Erfindung nicht auf diese spezielle Ausführungsform beschränkt ist, sondern aufgrund der Pluripotenz der Stammzellen bzw. Keimbahnzellen auf alle 3 Keimblatt-abgeleiteten Zelltypen, d.h. Endoderm, Mesoderm und Ektoderm und die sich hiervon ableitenden Zelltypen, anwendbar ist. Der Fachmann ist in der Lage, die Erfindung im Rahmen der beigefügten Ansprüche unter Berücksichtigung der nachfolgenden Beschreibung und seines allgemeinen Fachwissens zu variieren.

Erfindungsgemäß wird in embryonale Stammzellen, embryonale Keimbahnzellen und adulte Stammzellen die Information für zumindest ein Resistenzgen und für zumindest ein detektierbares Reportergen, kodierend ein nicht zellschädigendes fluoreszierendes Protein, eingeführt. Die Information für beide Gene kann auf einem oder zwei Vektoren verteilt vorliegen. Entscheidend ist, dass die Expression des Gens für das detektierbare, z.B. fluoreszierende Protein als auch für das Resistenzgen unter Kontrolle ein- und desselben Promotors steht.

Erfindungsgemäß werden die Promotoren unter zellspezifischen Promotoren und entwicklungsspezifischen Promotoren ausgewählt. Unter zell- bzw. gewebespezifischen Promotoren versteht man solche, die in spezifischen Zellpopulationen bzw. Geweben aktiv sind. Erfindungsgemäß werden Promotoren verwendet, die in Herzmuskelzellen (Kardiomyozyten) aktiv sind.

Beispiele für herzspezifische Promotoren sind: Nkx-2.5 (spezifisch für sehr frühe Kardiomyozyten bzw. mesodermale Vorläuferzellen, (Lints et al., 1993); Human-cardiac-a-Actin (spezifisch für Herzgewebe, (Sartorelli et al., 1990), MLC-2V (spezifisch für ventrikuläre Herzmuskelzellen (O'Brien et al., 1993) und WO-A-96/16163).

Beispiele für nicht-herzspezifische Promotoren sind: PECAM-1, FLK-1 (Endothel), Nestin (neurale Vorläuferzellen), Tyrosin-Hydroxylase-1-Promotor (dopaminerge Neurone), Smooth Muscle-α-actin, Smooth Muscle-Myosin (glatte Muskulatur), α1-Fetoprotein (Endoderm), Smooth muscle heavy chain (SMHC Minimal Promotor (spezifisch für glatte Muskelzellen, (Kallmeier et al., 1995).

Unter einem entwicklungsspezifischen Promotor sind Promotoren zu verstehen, die zu speziellen Zeitpunkten während der Entwicklung aktiv sind. Beispiele für derartige Promotoren sind der β-MHC Promoter, der während der Embryonalentwicklung im Ventrikel der Maus exprimiert wird und in der perinatalen Phase vom α-MHC-Promotor ersetzt wird. NKx2.5, ein Promotor während der frühen Mesoderm/Herzentwicklung, Atrial-Natriuretic-Factor, ein Marker des frühembryonalen Herzens mit Ausnahme des Schrittmacherzentrums, der auch zu späteren Entwicklungszeiten herunter reguliert wird, Flk-1, ein Endothel-spezifischer Promotor, der während der frühen Vasculogenese aktiv ist, Intron 2-Segment des Nestin-Gens, das in neuralen Vorläuferzellen (embryonalen Neuronen und Gliazellen) und adulten Gliazellen (teilweise noch teilungsfähig) exprimiert wird (Lothian and Lendahl, 1997).

Unter einem Promotor versteht man erfindungsgemäß einen DNA-Sequenzbereich, von dem aus die Transkription eines Gens gesteuert wird. Er umfaßt in einer Ausgestaltungsform zumindest eine Minimal-Sequenz, die stromaufwärts vom Startcodon gelegen ist und die Bindungsstelle für die RNA-Polymerase zur Initiation der Transkription umfaßt. Diese Minimal-Sequenz kann durch weitere funktionelle DNA-Abschnitte, insbesondere Enhancer, ergänzt werden. Es sind auch regulierende Elemente, die sich im Intronbereich befinden, und sich stromabwärts vom zu transkribierenden Gen befinden können, einsetzbar. Die Transkriptionsrate kann dann z. B. durch andere Enhancerelemente gesteuert werden, die per se keine Aktivität besitzen. Es sind auch Promotorkonstrukte einsetzbar, bei denen ein per se nicht konstitutiv aktives Element (heat shock protein enhancer) mit einem Enhancer-Segment des Gens, das aus dem Intron stammt, verwendet wird.

In einer weiteren Ausgestaltungsform der Erfindung werden entwicklungsspezifische Promotoren eingesetzt, die eine Selektion auf z.B. mesodermale Zellen ermöglichen. Einsetzbare Promotorelemente, welche die Transkription des Resistenzgens und des Gens für das detektierbare Protein steuern, sind Nkx2.5-, ANF- und Brachyuria-Promotoren. Nach Detektion der das detektierbare Protein exprimierenden Zellen, bei denen es sich z.B. bei Verwendung mesodermal-spezifischer Promotoren um mesodermale Zellen handelt, wird das dem Resistenzgen entsprechende Selektionsmittel zugegeben und auf die mesodermalen Vorläuferzellen selektioniert. Durch die von einem gemeinsamen Promotorelement gesteuerte Transkription des Gens für das detektierbare Protein und des Resistenzgens können hochspezifisch nicht differenzierte Zellen, z.B. embryonale pluripotente Stammzellen, abgetötet und damit die Möglichkeit einer späteren Tumorentstehung erheblich reduziert werden. Die so gewonnenen mesodermalen Zellen können dann in das entsprechende Gewebe implantiert werden und differenzieren dort weiter, beispielsweise nach Implantation in ein vorgeschädigtes Herzareal zu Herzzellen. Diese Vorgehensweise ermöglicht zum einen die Produktion großer Mengen aufgereinigter Vorläuferzellen und zum anderen nach Implantation eine weitere Differenzierung unter nativen Bedingungen.

In ähnlicher Weise ist es möglich, unter Verwendung endodermal oder ektodermal spezifischer Promotoren auf endodermale oder ektodermale Zellen zu selektionieren.

Beispiele für mesodermale Zellen sind alle Muskelzelltypen (Herzmuskel-, Skelettmuskel- und glatte Muskelzellen), hämatopoetische Zellen und Endothelzellen,. Beispiele für ektodermale Zellen sind Hautzellen, Neurone und Gliazellen; Beispiele für endodermale Zellen sind Epithelzellen des Magen-Darmtraktes.

Bei Einsatz der für die oben genannten Zelltypen spezifischen Promotoren erfolgt bei Verwendung des erfindungsgemäßen Verfahrens und der erfindungsgemäßen Zellen eine hochspezifische Entwicklung zu diesen endodermalen, ektodermalen bzw. mesodermalen Zellen und Geweben, wobei durch die von ein und demselben Promotor gemeinsam gesteuerte Expression des Reportergens und des Resistenzgens ein Höchstmaß an Sicherheit dadurch gewährleistet wird, daß einerseits die undifferenzierten pluripotenten embryonalen Stammzellen, andererseits aber auch andere Gewebetypen abgetötet werden.

Erfindungsgemäß kodiert das Reportergen für ein fluoreszierendes Protein. Derartige nichtzellschädigende fluoreszierende Proteine sind an sich bekannt. Gemäß der vorliegenden Erfindung kann das Green Fluorescent Protein (GFP) aus der Qualle Aequorea victoria (beschrieben in WO-A-95/07463, WO-A-96/27675 und WO-A-95121 191) und dessen Derivate "Blue GFP" (Heim et al., Curr. Biol. 6(2): 178-182 (1996) und Redshift GFP" (Muldoon et al.;Biotechniques 22 (1): 162-167 (1997)) verwendet werden. Besonders bevorzugt ist das Enhanced Green Fluorescent Protein (EGFP). Weitere Ausgestaltungen sind das gelb und das cyan fluoreszierende Protein (YFP, CFP). Weitere fluoreszierende Proteine sind dem Fachmann bekannt und können, solange sie die Zellen nicht schädigen, erfindungsgemäß eingesetzt werden. Der Nachweis der fluoreszierenden Proteine erfolgt durch an sich bekannte Fluoreszenz-Nachweisverfahren.

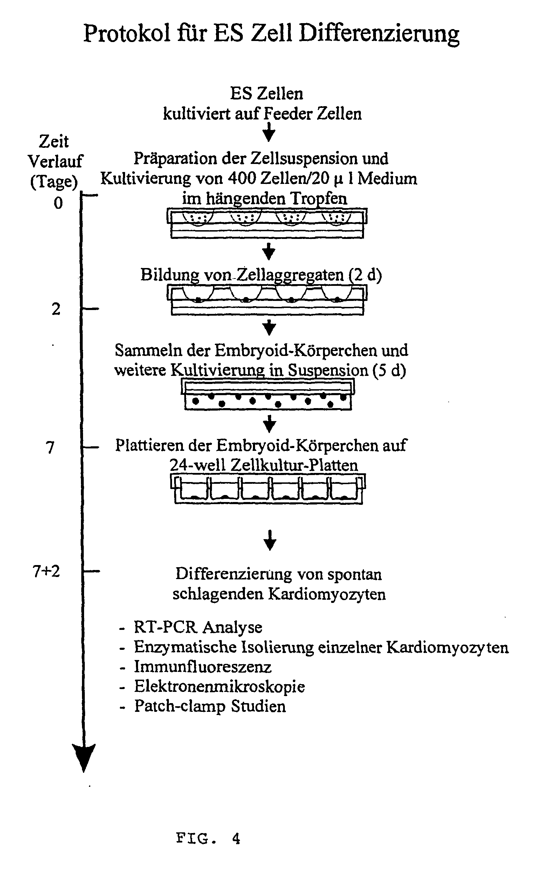

Die embryonalen oder adulten Stammzellen und die embryonalen Keimbahnzellen liegen in einer bevorzugten Ausgestaltungsform der Erfindung in Form von Aggregaten vor, die als embryoide Körperchen bezeichnet werden. Die Figur 4 zeigt ein Protokoll, um embryoide Körperchen zu erhalten. Die Herstellung erfolgt bevorzugt mit der Methodik des "hängenden Tropfens" oder durch Methylzellulosekultur (Wobus et al., Differentiation (1991) 48, 172-182).

Alternativ hierzu können auch die "spinner flasks" (Rührkulturen) als Kulturmethode eingesetzt werden. Hierfür werden die undifferenzierten ES Zellen in die Rührkulturen gebracht und nach einem etablierten Schema ständig durchmischt. Hierfür werden 10 Millionen ES Zellen in 150 ml Medium mit 20% FKS gebracht und konstant bei einer Geschwindigkeit von 20 r.p.m. gerührt, wobei regelmäßig die Richtung der Rührbewegung geändert wird. 24 Stunden nach Einbringen der ES Zellen werden zusätzlich 100 ml Medium mit Serum hinzugegeben und daraufhin jeden Tag 100 - 150 ml des Mediums ausgetauscht (Wartenberg et al., 2001). Unter diesen Kulturbedingungen können große Mengen an ES-Zell-abgeleiteten Zellen, u.a. Kardiomyozyten, Endothelzellen, Neurone u.s.w., je nach Zusammensetzung des Mediums, gewonnen werden. Entweder werden die Zellen mittels des Resistenzgenes noch in der Rührkultur bzw. nach Ausplattierung selektioniert.

Alternativ hierzu können die im hängenden Tropfen differenzierten EBs nicht plattiert, sondern einfach in Suspension gehalten werden. Selbst unter diesen Bedingungen konnte experimentell ein Forschreiten der Differenzierung festgestellt werden. Erstaunlicherweise zeigte es sich jedoch, daß die Applikation des Resistenzgens noch sehr viel schneller zu einem kompletten Absterben der nicht-Kardiomyozyten führte und die verbleibenden Kardiomyozyten daraufhin spontan zu schlagen beginnen. Dieser experimentelle Befund weist eindeutig darauf hin, daß Kardiomyozyten für das Überleben offensichtlich keine spezifischen Signale vom umgebenden Gewebe benötigen und ferner die Puromycin-selektionierten Kardiomyozyten funktionell intakt sind. Das Abwaschen der nicht-Kardiomyozyten ist ebenfalls deutlich erleichtert, da allein mit mechanischer Durchmischung bzw. niederkonzentrierter Zugabe von Enzymen (z.B. Kollagenase, Trypsin) eine Einzelzellsuspension mit leichtem Abwaschen der nicht-Kardiomyozyten erreicht wird.

Die embryonalen Stammzellen stammen von Säugetieren, insbesondere bevorzugt von Nagern, beispielsweise Mäusen, Ratten oder Kaninchen. Besonders bevorzugte ES-Zellen sind dabei D3-Zellen (Doetschman et al., 1985)(Doetschmann et al., J. Embryol. Exp. Morphol. 87, 27 (1985)), R1-Zellen (Nagy et al., PNAS (1995)), E14-Zellen (Handyside et al., Roux Arch. Develop. Biol. 198, 48 (1989)), CCE-Zellen (Bradley et al., Nature 309, 255 (1985)) (selbstverständlich sind auch andere ES Zellen einsetzbar, die bereits bekannt oder in Zukunft entwickelt werden) und P19-Zellen (dies sind Teratokarzinomabgeleitete Zellen mit eingeschränkten Eigenschaften (Mummery et al., Dev. Biol. 109, 402 (1985)).

In einer weiteren bevorzugten Ausführungsform werden embryonale Stammzellen von Primaten verwendet, wie sie beispielsweise von Thomson, J. A. et al., 1995 beschrieben wurden. Darunter versteht man auch menschliche Stammzellen, welche aber vom Schutzbereich ausgeschlossen sind.

In einer besonders bevorzugten Ausgestaltungsform werden humane embryonale Stammzellen verwendet. Die Gewinnung dieser embryonalen Stammzellen ist bereits etabliert (Thomson JA et al., 1998). Der Stammzellstatus kann durch die in vitro - Differenzierung in verschiedene Zelltypen und Propagation und Splittung über mehrere Passagen hinweg nachgewiesen werden.

Alternativ zu embryonalen Stammzellen eignen sich auch embryonale Keimbahnzellen (EG) (Shamblott MJ et al., 1998), die aus dem frühen Embryo gewonnen und in der Folgezeit wie embryonale Stammzellen kultiviert und differenziert werden können. Darunter versteht man auch menschliche Stammzellen, welche aber vom Schutzbereich ausgeschlossen sind.

Die Erfindung ist auch auf adulte Stammzellen anwendbar. Es wird hier auf die Literatur von Anderson et al., 2001, Gage, F.H., 2000 und Prockop, D.J., 1997, verwiesen, welche die Gewinnung und Kultur derartiger Zellen beschreibt.

Resistenzgene sind an sich bekannt. Beispiele hierfür sind Nukleosid- und Aminoglykosid-Antibiotika-Resistenzgene, z.B. Puromycin (Puromycin-N-Acetyltransferase), Streptomycin, Neomycin, Gentamycin oder Hygromycin. Weitere Beispiele für Resistenzgene sind Dehydrofolat-Reduktase, die eine Resistenz gegen Aminopterin und Methotrexat vermittelt, sowie Multidrug-Resistenzgene, die eine Resistenz gegen eine Anzahl von Antibiotika vermitteln, beispielsweise gegen Vinblastin, Doxorubicin und Actinomycin D. Besonders bevorzugt ist ein Konstrukt, welches eine Puromycin-Resistenz vermittelt. Die Ausdrücke Resistenzgen und Arzneistoff- bzw. Wirkstoff-Resistenzgen werden hier synonym verwendet und beziehen sich z.B. jeweils auf ein für eine Antibiotikum-Resistenz kodierendes Gen. Es können aber auch andere für Arzneimittel- bzw. Wirkstoff Resistenzen kodierende Gene eingesetzt werden, z.B. das DHFR-Gen. Anstatt der Resistenzgene können auch andere selektierbare Markergene eingesetzt werden, die eine spez. Selektion der das erfindungsgemäße Konstrukt enthaltenden Zellen ermöglichen und die, ohne das Überleben des Patienten zu beeinträchtigen, auch in vivo anwendbar sind. Geeignete Gene stehen hier dem Fachmann zur Verfügung.

Im ersten Ausführungsbeispiel liegen die Gene für das detektierbare Protein und das Resistenzgen auf zwei verschiedenen Konstrukten. Die Verwendung von zwei verschiedenen Vektoren, wobei auf dem ersten Vektor das Resistenzgen liegt und auf dem zweiten Vektor das Reportergen, beispielsweise EGFP, wobei beide von einem zell- bzw. gewebespezifischen oder entwicklungsspezifischen Promotor, beispielsweise dem α-MHC-Promotor kontrolliert werden, weist die in der vorliegenden Anmeldung aufgezeigten vielfältigen Vorteile auf, die sich für bestimmte Anwendungszwecke eignen. Weitere Versuche zeigten aber, daß dieses System überraschenderweise auch mit gewissen Nachteilen verbunden ist, nämlich mit der Bildung von zwar gegen das Resistenzgen resistenten Zellen, innerhalb derer sich jedoch Zell-Subklone befinden, die das Reportergen, z.B. EGFP, nicht exprimieren, also z.B. EGFP-negativ sind. Derartige Subklone können eine potentielle Quelle für Teratokarzinome darstellen, da, auch bei Einsatz des Antibiotikums, nicht alle nicht-spezifischen Zellen, also z.B. nicht-Kardiomyozyten, abgetötet werden. Dies könnte dazu führen, daß schnell proliferierende ES-Zellen potentiell überleben und Tumore bilden können.

In den durchgeführten Versuchen zeigt es sich, daß in der Tat EGFP-negative Zellen auch nach einer Puromycin-Exposition von bis zu 15 Tagen überleben können. Eine mögliche Ursache für diese Beobachtung liegt darin, daß die eingesetzten zwei Vektoren in einer Doppel-Transfektion in die Zellen eingebracht werden. Diese Vektoren integrieren dann zufällig im Wirtsgenom, teilweise an unterschiedlichen Stellen des nativen Genoms und können daher unter den Einfluß verschiedener Gene und ihrer Kontrollsequenzen geraten, die unterschiedliche Transkriptionsaktivitäten besitzen.

In einer weiteren Ausführungsform der Erfindung (Beispiel 2) wurden deshalb das Reportergen und das Resistenzgen auf einem Vektorkonstrukt unter Kontrolle eines Promotors angeordnet. Im vorliegenden Beispiel 2 wurden sowohl die Puromycinresistenzkassette (Pac) als auch das Reportergen EGFP unter gemeinsamer Kontrolle des gewebespezifischen Promotors α-MHC gebracht. Der große Vorteil dieses Systems liegt in der sehr geringen Inzidenz von resistenten Zellen, die nicht zell- oder gewebe- oder entwicklungsspezifisch sind. Beispielsweise ist die Wahrscheinlichkeit sehr gering, daß puromycinresistente Zellen auftreten, bei denen es sich nicht um Herzzellen handelt. Dies scheint darauf zurückzuführen zu sein, daß die Pac-Kassette und das EGFP-Gen nur an einer oder wenigen Lokalisationen des Wirtsgenoms integrieren und deshalb nicht dem Einfluß unterschiedlicher Aktivitätszustände der jeweiligen vor- bzw. nachgeschalteten Genstruktur unterliegen. Durch weitere Selektion der erhaltenen Klone ist es möglich, ein praktisch reines Zellsystem zu erhalten. Diese Evaluierung erfolgt unter Einsatz der EGFP-Expression. In diesem Zusammenhang wird nochmals darauf hingewiesen, daß es sich bei EGFP, α-MHC und Pac um beispielhafte Ausgestaltungen der Erfindung handelt. Der Fachmann kann hier unter Berücksichtigung der in der vorstehenden Anmeldung beschriebenen Alternativen Abänderungen vornehmen.

Das Einbringen des oder der Vektor-Konstrukte in die embryonalen Stammzellen erfolgt in an sich bekannter Weise, beispielsweise durch Transfektion, Elektroporation, Lipofektion oder mit Hilfe viraler Vektoren.

Zur Selektion auf stabil transfizierte ES-Zellen enthalten die Vektor-Konstrukte ein weiteres selektierbares Markergen, welches beispielsweise eine Resistenz gegen ein Antibiotikum vermittelt, beispielsweise Neomycin. Selbstverständlich können auch andere, an sich bekannte Resistenzgene eingesetzt werden, beispielsweise die weiter oben i.V. mit dem für das fluoreszierende Protein kodierenden Gen beschriebenen Resistenzgene. Das zur Selektion auf stabil transfizierte ES-Zellen eingesetzte Selektions-Gen steht unter Kontrolle eines anderen Promotors als dasjenige, welcher die Kontrolle der Expression des detektierbaren Proteins reguliert. Häufig werden konstitutiv aktive Promotoren eingesetzt, beispielsweise der PGK-Promotor.

Die Verwendung des zweiten Selektionsgens ist wichtig, um überhaupt die erfolgreich transfizierten Klone (Effizienz relativ niedrig) identifizieren zu können. Andererseits würde eine erdrückende Mehrzahl von nicht transfizierten ES-Zellen vorliegen und bei der Differenzierung z.B. keine EGFP positiven Zellen erkannt werden.

Nach Transfektion werden die Konstrukte stabil in die native DNA integriert. Nach Aktivierung intrazellulärer Signale, die entweder zellspezifischer und/oder entwicklungsspezifischer Natur sind, wird der Promotor aktiviert und es werden sowohl das detektierbare Protein als auch das (erste) Resistenzgen exprimiert. Es können damit nicht nur ES-Zellen z.B. anhand ihrer Fluoreszenz-Emission unter Fluoreszenzanregung erkannt werden, sondern es kann gleichzeitig und hoch-spezifisch auf diejenigen Zellen selektiert werden, die unter Kontrolle des zellspezifischen und/oder entwicklungsspezifischen Promotors stehen. Mit dieser als elegant zu bezeichnenden Methode ist eine hohe Anreicherung spezifischer Zellen möglich, die in einem speziellen Entwicklungszustand aktiv oder die für spezifische Gewebe typisch sind. Ein besonders wichtiges Beispiel ist hier die Anreicherung von aus ES-Zellen stammenden Kardiomyozyten. Beispielhaft werden die nachfolgenden Vorteile genannt:
1. Die Kontrolle sowohl des Resistenzgens als auch des entwicklungsspezifischen und/oder zellspezifischen Gens unter ein und demselben Promotor garantiert eine effiziente und schnelle Selektion der beispielsweise gewebespezifischen Zellen, also beispielsweise der Herzzellen. Anhand von FACS-Analysen konnte gezeigt werden, dass beinahe 99% aller nicht herzspezifischen Zellen abgetötet wurden. Dieser hohe Reinheitsgrad für einen spezifischen Zelltyp innerhalb einer hoch heterogenen Zellpopulation von embryoiden Körperchen ist auch ein geeignetes Werkzeug nicht nur für pharmakologische Tests auf toxische Substanzen, zur Wirkstofftestung, embryotoxikologische Effekte, Austestung von Faktoren zur Zellproliferation und Differenzierung, sondern eröffnet auch die Möglichkeit, hochreine Zellpopulationen für therapeutische Anwendungen zum Ersatz von Geweben bereit zu stellen bzw. die Generation von Gewebestücken in vitro (Bioengineering) zu generieren.
2. Obwohl das erfindungsgemäß bevorzugt angewandte Differenzierungsverfahren mit dem "hängenden Tropfen" Zellpopulationen mit relativ stabilen Differenzierungscharakteristika bei Plattierung ermöglicht, weisen embryoide Körperchen gleichwohl sehr deutliche Unterschiede zum Zeitpunkt der Initiation der Differenzierung, u.a. auch des spontanen Schlagens, auf. Durch die Expression z.B. des Fluoreszenzgens erhält man eine zuverlässige Information über die Initiation der Differenzierung, z.B. der Kardiomyogenese, und die Zugabe des Selektionsmediums kann zeitlich abgestimmt nach Initiation der Transkription vom zellspezifischen oder entwicklungsspezifischen Promotor erfolgen. Die erfindungsgemäße Kombination eines Gens, welches für ein detektierbares, z. B. für ein fluoreszierendes Protein, kodiert, mit einem Selektionsgen, wobei beide Gene unter Kontrolle eines Promotors stehen, ermöglicht somit eine genaue zeitliche Abstimmung der Zugabe des Selektionsmediums in Abhängigkeit vom Differenzierungsstadium der Zellen, wobei das Differenzierungsstadium über die Expression des fluoreszierenden Proteins vom Experimentator feststellbar ist. Unter in vivo Bedingungen ist die Verwendung des Reportergens nicht kritisch, da es sowieso nicht identifiziert werden könnte. Es ist aber von Bedeutung bei der experimentellen Austestung (sehr wichtig zur Etablierung von Aufreinigungs-, aber auch operativen Verfahren) der Methode, aber evtl. aufgrund der potentiellen Antigenität für therapeutische Zwecke nicht einzusetzen. Alternativ, besonders für therapeutische Zwecke, ist die Verwendung eines transgenen Epitops, das keine Anbindung an intrazelluläre Signalkaskaden besitzt (z.B. CD8 oder CD4), unter Kontrolle des zell- bzw. gewebespezifischen Promotors geeignet. Mit Hilfe dieser Technik können nach der Puromycin- Anreicherung hochaufgereinigte Kardiomyozytenpräparationen mittels MACS-Sortierung nach Anreicherung mit z.B. Percoll Gradient erhalten werden; ferner können in vivo und in vitro die transgenen Zellen mittels anti-CD8 (anti-CD4) fluoreszent konjugierten Zell-Oberflächenantikörpern leicht identifiziert werden. Gleichzeitig kann auch die höchstmögliche quantitative Anreicherung des gewünschten Zelltyps erreicht werden. Eine zufällige Zugabe des Selektionsmediums, unabhängig von der Information über die Zelldifferenzierung, würde zu einer vorzeitigen Zerstörung der Vorläuferzellen oder nur zu einer geringen Anzahl terminal differenzierter Zellen führen.
3. Es ist davon auszugehen, dass die Differenzierung von ES-Zellen zu spezifischen Zelltypen, insbesondere in der natürlichen Umgebung des jeweiligen Organs, besonders effizient verläuft, da in der Organumgebung weitere Faktoren vorliegen, die die gewebespezifische Differenzierung der ES-Zellen vorantreiben. In der Tat konnten wir in unseren Transplantationsversuchen zeigen, dass ohne eine Gewebsschädigung (Abwesenheit von Differenzierungsfaktoren) kein Einwachsen und keine Differenzierung transplantierter embryonaler Herzmuskelzellen zu beobachten ist. Ferner kann nach der Transplantation in ein kryoinfarktiertes Areal eine deutlich gesteigerte Herzmuskelgeneration unter Verwendung undifferenzierter embryonaler Stammzellen beobachtet werden (in vitro nur 3-5%, in vivo wesentlich effektiver, aber mit einer Generation von Tumoren). Aufgrund der hohen Sensivität von ES-Zellen ohne das Resistenzgen gegen Antibiotika kann das erfindungsgemäße Verfahren dazu benutzt werden, die erfindungsgemäß bereit gestellten transgenen ES-Zellen in das jeweilige Organ in vitro oder in vivo einzubringen, in welchem die hoch effiziente Differenzierung, beispielsweise zu Herzzellen, stattfindet. Nach mehreren Wochen wird dann das Selektionsmedium zugegeben, und alle von den ES-Zellen stammenden Zellen mit Ausnahme der das Resistenzgen tragenden werden systematisch abgetötet. Bei dieser Vorgehensweise ist eine effizientere Geweberegeneration ohne das damit verbundene Risiko einer Tumorentstehung zu erwarten. Entscheidend für das hier entwickelte System ist, daß das Antibiotikaresistenzgen und das Reportergen unter Kontrolle desselben Promotors stehen. Der Grund hierfür liegt darin, daß das Reportergen den Zeitpunkt des Beginns der zellspezifischen bzw. entwicklungsspezifischen Differenzierung, z.B. der Herzdifferenzierung anzeigt; z.B. ist ein Großteil der frühen Herzzellen bereits gebildet und noch proliferativ. Zu diesem Zeitpunkt wird nun auch das Antibiotikaresistenzgen gebildet und somit alle Zellen nach Zugabe des Antibiotikas abgetötet, abgesehen von den Zellen, die das Resistenzgen exprimieren, z.B. also den Kardiomyozyten. In der DE 19727962 wurden unterschiedliche Promotoren verwendet, so dass diese Synchronisation nicht gegeben und deshalb die Selektion ineffizient war.
   Anstatt einer Doppeltransfektion könnte auch ein Vektorkonstrukt mit einer IRES gebaut werden, in der ein und derselbe Promotor, z.B. der α-MHC Promotor, das Reportergen und das Antibiotikaresistenzgen treibt und somit eine einfache Transfektion genügt.

Ein wichtiges Ziel der Erfindung liegt natürlich nicht nur in der in vitro-, sondern insbesondere in der in vivo-Anwendbarkeit der durch das erfindungsgemäße Verfahren erhältlichen differenzierten Zellen, insbesondere von Herzzellen. Um auszuschließen, daß bei einer Transplantation beispielsweise pluripotente Stammzellen oder Keimbahnzellen, die sich zu Tumorzellen entwickeln könnten, in den Patienten gelangen, können in einer Ausführungsform der Erfindung die Zellen durch Überexpression, beispielsweise durch Verwendung eines Oct-4 Promotors, noch sensibler für das Resistenzagenz gemacht werden. Hierdurch wird die Wahrscheinlichkeit, daß pluripotente Zellen den Angriff durch das Resistenzagens überleben, weiter verringert.

In einer weiteren Ausführungsform der Erfindung können die Zellen zusätzlich so manipuliert werden, daß spezifische Gewebe nicht gebildet werden. Dies kann beispielsweise durch Einfügen von Repressorelementen, z.B. eines Doxizyklininduzierbaren Repressorelements, erfolgen. Hierdurch könnte eine mögliche Kontamination der gewünschten differenzierten Zellen mit pluripotenten, potentiell tumorogenen Zellen ausgeschlossen werden.

In einer weiteren Ausführungsform kann durch die geeignete Wahl eines Promotors, beispielsweise des chicken β-Aktin-Promotors, auf Zellen mit einer hohen Teilungsrate selektiert werden und über diesen Weg die Möglichkeit eines Überlebens pluripotenter Zellen weiter verringert werden.

In einer bevorzugten Ausführungsform wurden zwei Arten von Vektoren verwendet, um embryonale Stammzellen stabil zu transfizieren und um Herzzellen spezifisch aus einer differenzierenden Kultur von embryonalen Stammzellen zu selektieren:
1. das Herz-α-MHC-Promotor gesteuerte Resistenzgen für Puromycin (α-MHC-Pur);
2. das Herz-α-MHC-Promotor gesteuerte Gen des verstärkten Grün Fluoreszierenden Proteins (enhanced Green Fluorescent Protein=EGFP)(α-MHC-EGFP).

Das Neue der vorliegenden Erfindung besteht in der kombinierten Anwendung sowohl eines Resistenzgens (z.B. Pur) als auch z.B. eines live Fluoreszenz-Reportergens (z.B. EGFP) unter Kontrolle desselben, bevorzugt herzspezifischen Promotors (z.B. α-MHC). Ein derartiger Ansatz zeigt eine Kombination der nachfolgenden Vorteile, die die genetische Selektion, z.B. der von ES abstammenden Herzmuskelzellen, erleichtern:
i) Überwachen der Differenzierung von embryonalen Stammzellen, z.B. der Herzdifferenzierung von sehr frühen Entwicklungsstadien ab durch Nachweis einer spezifischen, z.B. herzspezifischen Fluoreszenz (Kolossov et al., 1998).
ii) Optimierung der Zeit für den Beginn einer Arzneistoff-Anwendung durch Definition der Fluoreszenz als Indikator einer z.B. α-MHC-Promotor-Aktivität, die das Resistenzgen steuert.
iii) Visuelle Kontrolle des Prozesses der Arzneistoff-Selektion durch live-Überwachung bzw. -Monitoring des Verhältnisses zwischen fluoreszenten und nicht fluoreszenten Zell-Fraktionen. Durchführbarkeit einer quantitativen Schätzung des Spiegels einer spezifischen Zelltyp- Anreicherung mittels Fluorescence Activated Cell Sorting (FACS).
iv) Die bevorzugte Verwendung des Pur-Gens unter Kontrolle eines bevorzugt herzspezifischen Promotors ermöglicht eine hocheffektive herzspezifische Selektion durch Puromycin sowohl in adhärenten als auch in Suspensions-Kulturen differenzierender ES-Zellen, weil Puromycin eine schnellere und stärkere toxische Wirkung auf nicht resistente Zellen als andere bekannte Selektionsmittel, wie beispielsweise G418 und Hygromycin, aufweist.
   Die vorliegende hocheffiziente und sehr schnelle Selektion mit Puromycin im Gegensatz zu anderen Antibiotikaresistenzgenen war überraschend. Ferner war es für ES Zellen eine völlig unbekannte Beobachtung.
v) Die Möglichkeit der Überwachung des Schicksals eingefügter selektierter Zellen nach Transplantation durch bloße Anwendung des z.B. EGFP-Fluoreszenznachweises. Dies ist von grundlegender Bedeutung zur Etablierung neuer Operationstechniken.

Die Erfindung beinhaltet mehrere Aspekte, die unter Berücksichtigung des Standes der Technik nicht mit einer vernünftigen Aussicht auf Erfolg zu erwarten waren.
1. Zuerst überraschte die Einfachheit, mit der die ES Zellen auch doppelt transfiziert werden konnten. Unsere Versuche zeigten, daß in den meisten transfizierten Klonen eine effektive Transfektion mit beiden Konstrukten stattgefünden hatte.
2. Ferner zeigte sich kritisch für die Effizienz der Antibiotikaresistenz, daß das Selektionsagens während der frühen Phase der Differenzierung, insbesondere der Differenzierung von Herzzellen, zugegeben wird. Hierdurch wird offensichtlich die Effizienz der z.B. Kardiomyogenese in vitro erhöht, höchstwahrscheinlich weil die umgebenden Zellen negative Signale freisetzen. Als frühe Phase sind besonders bei der hängenden Tropfenmethode mit Plattierung 2-4 Tage nach der Plattierung gemeint, einem Stadium in dem in Bezug auf Proliferation (Zellen sind noch proliferativ) sowie Ionenkanalexpression (If Kanal ist noch in allen Kardiomyozyten exprimiert, alle Zelltypen auch Ventrikelzellen exprimieren diesen Ionenkanal und schlagen spontan) und deren Regulation (basale Hemmung des L-Typ Ca²⁺ Stroms mittels muskarinergen Agonisten über das Stickstoff-Monoxid System) noch frühe Muster vorzufinden sind.
3. Überraschend war auch die hocheffiziente Aktion von Puromycin, das innerhalb von 12-24 Stunden zu einer 99% Abtötung aller nicht-Kardiomyozyten im EB führt.
4. Ein entscheidender Vorteil der vorliegenden Erfindung ist die Möglichkeit einer Selektion auch an nicht plattierten EBs bzw. in den Rührkulturen, da hier die abgetöteten Zellen unproblematisch abgewaschen und somit erstmals reine zelltypspezifische Kulturen aus ES Zellen gewonnen werden können. Teilweise wird die Elimination der nicht vitalen Zellen durch enzymatische Verdauung (z.B. Trypsin, Kollagenase) verbessert. Die Effizienz dieser Methode konnte ferner dadurch bestätigt werden, daß Kardiomyozyten in nicht plattierten EBs erneut als Zellverband spontan zu kontrahieren beginnen.

In einer weiteren Ausführungsform der Erfindung werden die embryonalen Stammzellen stabil mit zwei Sätzen von Vektor-Selektionssystemen transfiziert. Der erste Vektor enthält die Information für ein erstes nicht zellschädigendes detektierbares, z.B. fluoreszierendes Protein und/oder für ein erstes Resistenzgen, und beide Gene stehen unter Kontrolle eines ersten zellspezifischen oder entwicklungsspezifischen Promotors, der jeweils in funktioneller Weise mit den genannten Genen verbunden ist. Ein zweiter Vektor enthält die Information für ein zweites nicht zellschädigendes, detektierbares, z.B. fluoreszierendes Protein und/oder ein zweites Resistenzgen, und beide Gene liegen unter Kontrolle eines zweiten zellspezifischen oder entwicklungsspezifischen Promotors, der wiederum in funktioneller Weise jeweils mit diesen Genen verknüpft ist. Alternativ zur Elektroporation kann ein hocheffiziente Transfektion auch mit Viren sowie mit Lipofektion vorgenommen werden.

Besonders erwähnenswert im Zusammenhang mit einer erfolgreichen Transplantation am Herzen ist die in vitro Selektion mesodermaler Vorläuferzellen. Diese Zellen werden entsprechend obigem Schema unter bevorzugt Brachyuria, Nkx2.5 und ANF Promotor-Schaltelementen exprimierten Fluoreszenz- und Resistenzgenen selektioniert und daraufhin transplantiert. Anstatt des Fluoreszenzgens sind natürlich auch Gene der oben beschriebenen anderen detektierbaren Proteine einsetzbar. Dieses Schema ist ideal dazu geeignet, große Menge von aufgereinigten Vorläuferzellen zu produzieren, die ohne Gefahr z.B. nach Implantation ins geschädigte Myokard unter nativen Differenzierungsfaktoren *in situ* zu Herzzellen differenzieren.

Ferner eignet sich dieser Ansatz in idealer Weise dazu, unterschiedliche Wirkstoffe/Differenzierungsfaktoren *in vitro* auszutesten, die mesodermale Vorläuferzellen in die unterschiedlichen spezialisierten Zelltypen (u.a. immunologische Zellen, glatte- und Skelettmuskelzellen sowie Endothelzellen) differenzieren. Somit eignet sich dieses System ideal zur Testung von Differenzierungsfaktoren, pharmakologischen und unterschiedlichen Wirkstoffen (u.a. toxikologischen Substanzen, Umweltgiften, Gebrauchschemikalien, zum Test auf teratogene/embryotoxikologische Effekte und zur Wirkstoffkunde).

Ferner wurde neben der *in vitro* Differenzierung und Selektion ein völlig neues Schema zur Gewebewiederherstellung etabliert. Hierbei wird einerseits der Vorteil ausgenutzt, daß im geschädigten Gewebe (z.B. im Herzinfarktareal) native Faktoren freigesetzt werden, die die Herzzelldifferenzierung positiv beeinflussen. Deshalb werden z.B. transgene embryonale Stammzellen generiert, in denen einerseits z.B. insbesondere das Puromycin-Resistenzgen unter Kontrolle z.B. des α-MHC Promotors steht (α-MHC-Puromycin). Um die Möglichkeit der Tumorentstehung auszuschließen, wird zusätzlich das Poxvirus getriebene tk-Element verwendet. Deshalb werden die embryonalen Stammzellen mit einem ubiquitär exprimierten Promotor (z.B. chicken β actin Promotor) und dem anti-tk Element unter Kontrolle des α-MHC Promotors triple transfiziert. Daraufhin werden diese transgenen, undifferenzierten ES Zellen in das geschädigte Herzareal gespritzt. Die intrinsischen Faktoren begünstigen eine hocheffiziente Herzentwicklung der ES Zellen *in vivo* im Gegensatz zu der *in vitro* Differenzierungskapazität. Nach 14-21 Tagen werden selektiv alle nicht-Kardiomyozyten mittels der kombinierten systemischen Gabe des Resistenzagens, z.B. Puromycin und des Virostatikums Gancyclovir, selektioniert. Durch diese kombinierte Selektion wird das potentielle Überleben undifferenzierter ES Zellen und die Gefahr der Tumorogenität vermieden. Ferner wird ein wesentlich effizienterer Herzmuskelaufbau erzielt.

Nachfolgend wird die Erfindung anhand von Ausführungsbeispielen und der beiliegenden Figuren näher erläutert. Die Figuren zeigen:
Fig. 1: Kombinierte Transmission/Fluoreszenz-Lichtmikroskopie-Bilder von ausplattierten EBs, die von pαMHC-Pur transgenen ES-Zellen abstammten, am 10.(A), 11. (B), 12. (C) und 14. (D) Tag der Entwicklung nach 1, 2, 3 beziehungsweise 5 Tagen der Puromycin-Behandlung.
Fig. 2: Kombinierte Transmission/Fluoreszenz-Lichtmikroskopie-Bilder einer Suspensionskultur von pαMHC-EGFP/pαMHC-Pur EBs am 19. Tag der Entwicklung nach 10 Tagen Puromycin-Behandlung.
Fig. 3: (A) FACS-Profil der dissoziierten, 16 Tage alten EBs, die von pαMHC-EGFP transgenen ES-Zellen abstammten. Alle EBs enthielten große schlagende und fluoreszierende Herzmuskelzell-Cluster. EGFP positive Zellen (M1) bilden weniger als 1% der Gesamtzellpopulation.
   (B) FACS-Profil der dissoziierten 22 Tage alten EBs, die von kotransfizierten pαMHC-EGFP und pαMHC-Pur ES-Zellen abstammten, nach 13 Tagen Puromycin-Behandlung. EGFP positive Zellen (m1) bilden 42 - 45% der Gesamtzellpopulation.
Fig. 4: Protokoll zur Gewinnung von embryoiden Körperchen

### Beispiel 1

### MATERIALIEN UND METHODEN.

### Vektoren.

Der ein regulatorisches 5,5 kb Fragment des Maus α-MHC-Gens enthaltende Vektor wurde von Dr. J. Robbins (Children Hospital Medical Center, Cincinnati, USA) bereitgestellt (Gulick et al., 1991).

Das Fragment wurde aus dem Vektor mit BamHI und SalI ausgeschnitten, mit Blunt-Ends versehen und in die SmaI-Stelle der multiplen Klonierungsstelle des pEGFP-1 Vektors kloniert (enthält die kodierende Sequenz für EGFP, die verstärkte Version von GFP und die Neo-Kassette für die G418-Resistenz) (KLONTECH Laboratories, Palo Alto, CA, USA). Die richtige "Schwanz-an Kopf" (tail-to-head) -Orientierung des Promotors zur kodierenden Sequenz von EGFP im sich ergebenden Vektor wurde überprüft und durch EcoRI-Restriktion bestätigt.

Der kodierende Teil des Pur-Gens (HindIII-SalI-Fragment) wurde in pαMHC-EGFP anstatt der durch BamHI-AflII ausgeschnittenen EGFP-kodierenden Sequenz blunt-ligiert (Ligation stumpfer Enden). Die richtige Ausrichtung beziehungsweise Orientierung im sich ergebenden Vektor pα-MHC-Pur wurde durch SmaI und ClaI-StuI-Restriktionen bestätigt.

### Zellkultur, Transfektions- und Selektions-Verfahren.

Alle Stadien der Vermehrung und der Selektion von ES-Zellklonen wurden in ES-Zell-Vermehrungsmedium durchgeführt das aus folgendem bestand: Glucosereiches DMEM Medium ergänzt mit:
nicht essentiellen Aminosäuren (0,1 mM), L-Glutamin (2mM), Penicillin und Streptomycin (jeweils 50ug/ml), β-Mercaptoethanol (0,1 mM), LIF (ESGRO^{™}) (500u/ml), fötales Kälberserum (FCS) (15% V/V).

Beide Vektoren, pαMHC-EGFP und pαMHC-Pur, wurden durch HindIII-Restriktase vor Cotransfektion durch Elektroporation der ES-Zellen (D3 Linie) linearisiert. Elektroporationsbedingungen:
Zellen: 4 bis 5 x 10⁶ in 0,8 ml PBS (Ca²⁺, Mg²⁺ frei) Vektor-DNA: 20-40µg;
Elektroporations-Küvette: 0,4cm (Bio-Rad Laboratories, Hercules, CA, USA);
Elektroporationsgerät: Gene Pulser^{™} (Bio-Rad Laboratories); Elektrische Impulsbedingungen: 240V, 500µF.

Nach dem elektrischen Impuls wurde die Zellsuspension 20 Minuten lang in Eis gekühlt und dann mit einem G418-resistenten Fibroblasten-Feeder Layer in 10 ml ES-Zellvermehrungs-Medium auf die 10 cm Gewebs-Qualität Petrischale übertragen. 2 Tage später wurde Geneticin G418 (GibcoBRL), 300 µg/ml zur Selektion von G418-resistenten Zellen zugesetzt. Das Medium mit G418 (300 µg/ml) wurde jeden zweiten Tag ausgetauscht. Nach 8 - 10 Tagen Selektion erschienen die Arzneistoff-resistenten Kolonien. Die Kolonien wurden herausgenommen, getrennt in 0,1% Trypsin/EDTA-Lösung trypsiniert und auf 48 Well-Platten mit G418 resistenter Fibroblasten-Feeder Layer in ES-Zellvermehrungs-Medium und G 418 (300µg/ml) ausplattiert. Nach 2 - 4 Tagen Wachstum wurden ES-Zellklone nachfolgend trypsinisiert und auf 24 Well-Platten und darauf auf 5 cm Gewebs-Petrischalen vermehrt. G418 (300 µg/ml) und G418 resistente Fibroblasten-Feeder Layer lagen in allen Stadien der ES-Zellklon-Vermehrung vor.

### Differenzierung von ES-Zellen und herzspezifische Selektion.

Alle Schritte der Differenzierungsvorschrift wurden im "Differenzierungsmedium" durchgeführt, das aus allen Bestandteilen des vorstehend erwähnten "ES-Zell-Vermehrungsmediums", ausschließlich LIF, bestand und bei dem 15% FCS durch 20% FCS ersetzt waren. Nach der Vermehrung wurden die ausgewählten G418 resistenten ES-Klone trypsinisiert und in "Differenzierungsmedium" zu einer Endkonzentration von 0,020 bis 0,025 x 10⁶ Zellen/ml resuspendiert. Anschließend wurden "hängende Tropfen" durch Anordnen von 20µl dieser Suspension (400 bis 500 Zellen) an den Deckeln von Bakterienpetrischalen (Greiner Labortechnik, Deutschland) gebildet. Nach 2 Tagen Inkubation bei 37°C und 5% CO₂ bildeten die ES-Zellen die Aggregate oder "embryoiden Körper", die in die bakteriellen Petrischalen mit "Differenzierungsmedium" ausgewaschen und für zusätzliche 5 Tage inkubiert wurden. Darauf wurden die embryoiden Körperchen getrennt auf mit Gelatine vorbehandelten 24 Well Gewebs-Qualitäts-Platten in "Differenzierungsmedium" plattiert. In parallelen Experimenten wurde eine Anzahl von embryoiden Körperchen in Suspension gelassen, wo sie ebenfalls wie die plattierten behandelt wurden.

In allen Wachstums-, Differenzierungs- und Arzneistoff-Selektions-Stadien wurden EBs unter dem Fluoreszenzmikroskop unter Verwendung eines FITC Filtersets (Zeiss, Jena, Deutschland) überwacht.

Bei typischen Experimenten wurde die Anwendung des selektiven Arzneistoffes Puromycin (1 - 2 µg/ml) am Tag 9 - 10 der Entwicklung begonnen, wenn die erste EGFP-Fluoreszenz überwacht wurde. Das Medium mit der Wirksubstanz wurde alle 2 - 3 Tage ausgetauscht.

### FACS-Analyse

Für eine FACS-Analyse wurden 10 bis 20 embryoide Körperchen aus unterschiedlichen Entwicklungs- und Selektionsstadien mit PBS gewaschen und dann durch Trypsin-Behandlung für 2-3 Minuten (120µl Trypsin/EDTA-Lösung) zu einer einzigen Zellsuspension dissoziiert. Daraufhin wurden 1 ml DMEM + 20% FCS der einzigen Zellsuspension zugesetzt. Nach Zentrifugierung (1000 UpM) für 5 Minuten wurden die Zellen in 0,5 - 1,0 ml PBS, das Ca²⁺ (1 mM) und Mg²⁺ (0,5 mM) enthielt, resuspendiert.

Die GFP Expression durch von embryonalen Stammzellen abstammende Zellen unterschiedlichen Alters wurde auf einem FACSCa-libur^{™} Durchflußzytometer (Becton Dickinson, BRD) untersucht, das mit einem 488 nm Argonionen-Laser (15 mW) ausgestattet war. Die Zellen wurden zu einer Konzentration von 5 x 10⁵ Zellen/ml in PBS (pH 7,0, 0,1% BSA) resuspendiert und dann auf dem FACSCalibur^{™} mit einem Minimum von 10.000 lebensfähigen Zellen untersucht, die für jede Probe gewonnen wurden. Die emittierte Fluoreszenz von GFP wurde bei 530 nm (FITC-Bandfilter) gemessen. Das live Gating wurde durch Zusatz von Propidiumiodid (2µg/ml) zu den Proben unmittelbar vor der Messung durchgeführt. Nekrotische Zellen mit einer positiven Propidiumiodid (PI) Färbung (585 nm Bandfilter) zeigten höhere Seiten-Streuungs(SSC)-Signale im Vergleich zu den lebensfähigen PI-negativen Zellen. Nicht lebensfähige Zellen wurden aus der nachfolgenden Untersuchung ausgeschlossen, indem Zellen mit niedrigen SSC-Signalen durchgelassen wurden. Nicht transfizierte ES-Zellen der Zelllinie D3 wurden für Negativkontrollen verwendet. Untersuchungen wurden unter Verwendung der CellQuest^{®} Software (Becton Dickinson) durchgeführt.

### ERGEBNISSE

ES-Zellen, die sowohl bezüglich pαMHC-EGFP als auch pαMHC-Pur-Vektoren transgen waren, wurden vermehrt und in der Herz-Differenzierungs-Vorschrift verwendet. Alle getesteten Klone zeigten im ES-Zellstadium und nach Bildung von EBs bis zum Tag des Plattierens (7 Tage nach der Bildung von "hängenden" Tropfen) keine mikroskopisch nachgewiesene EGFP-Fluoreszenz. Am ersten bis zweiten Tag nach dem Ausplattieren (8 - 9 Tage alte EBs) erschienen die ersten EGFP-fluoreszierenden Gebiete, die üblicherweise einen Tag später spontan zu schlagen begannen. Bemerkenswerterweise zeigte die überwiegende Mehrheit von EB-Zellen außerhalb der schlagenden Cluster kein mikroskopisch nachweisbares Fluoreszenzniveau, was eine hohe Gewebsspezifität der EGFP-Expression während der ES-Zellkardiomyogenese zeigt.

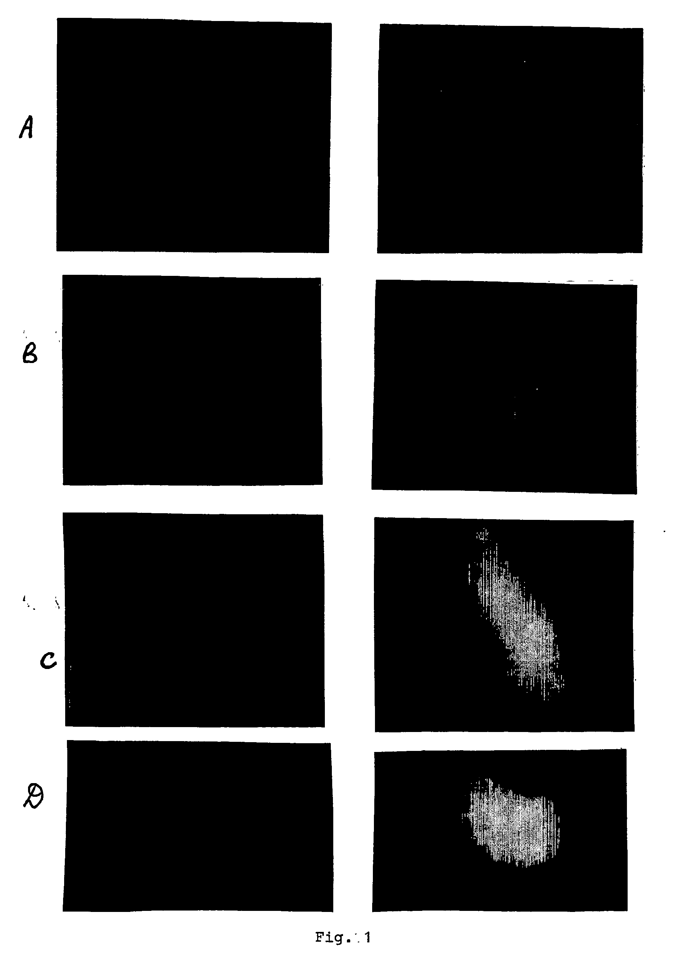

Nach Anwendung von Puromycin (typischerweise beginnend am Tag 9 - 10 der Entwicklung) wurden die ersten signifikanten Veränderungen in der Morphologie der ausplattierten EBs bereits innerhalb von 12 Stunden (mittels Langzeitbeobachtungssystem) am nächsten Tag nachgewiesen: Der Zellauswuchs, der die schlagenden Cluster der EGFP-fluoreszierenden Zellen umgab, hatte dramatisch abgenommen und die Schlagintensitäten der vom umgebenden Auswuchs befreiten Cluster hatte sich in unerwarteter Weise intensiviert (Fig. 1A). Während der nächsten wenigen Tage schritten diese Veränderungen fort und zeigten sowohl ernsthafte Zerstörung nicht fluoreszierender Zellmassen als auch eine Kompaktierung fluoreszierender Herz-Cluster mit intensiven kontraktilen Aktivitäten (Fig. 1B,C). Bereits am Tag 1 der Puromycin-Behandlung hatten sich einige der embryoiden Körperchen visuell der sie umgebenden nicht fluoreszierenden Zellen entledigt und sahen wie isolierte, schlagende und fluoreszierende Cluster aus (Fig. 1D). Sogar nach 4 Wochen der Entwicklung und nach 18 Tagen der Puromycin-Behandlung zeigten derartige isolierte Kluster noch eine intensive kontraktile Aktivität, wohingegen bei ihren unbehandelten Gegenstücken typischerweise eine derartige Aktivität am 17.-20. Tag der Entwicklung zum Stillstand kam.

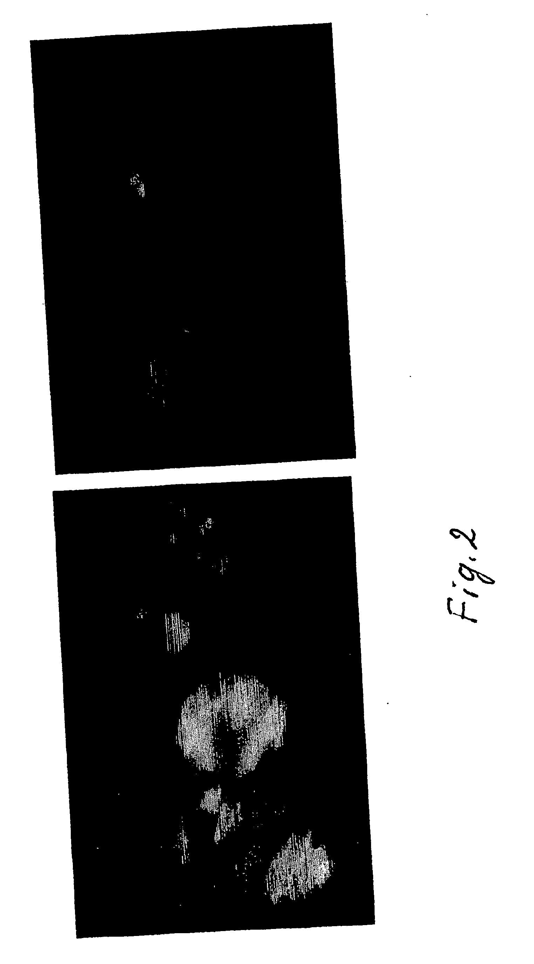

Sowohl die erhöhte EGFP-Fluoreszenz als auch die lang anhaltende kontraktile Aktivität wurden bei Puromycin-behandelten embryoiden Körperchen in Suspensions-Kultur im Vergleich mit unbehandelten Gegenstücken überwacht. Nach mehr als drei Wochen der Entwicklung und zwei Wochen der Puromycin-Behandlung waren Suspensionen von embryoiden Körperchen reichlich mit intensiv fluorenszierenden und kontraktilen embryoiden Körperchen versehen, von denen einige sichtbar als insgesamt schlagende fluoreszierende Cluster vorlagen (Fig. 2).Diese Befunde belegen eindeutig, daß Kardiomyozyten ohne die umgebenden Zellen weiter differenzieren und am Leben gehalten werden können. Ferner zeigt das spontane Schlagen die funktionelle Integrität der selektionierten Herzmuskelzellen auf. Von entscheidendem Vorteil war jedoch auch die Schnelligkeit der Puromycin Selektion, die innerhalb von 12-24 Stunden nach Applikation zu einer 99% Vernichtung aller nicht-Kardiomyozyten führt.

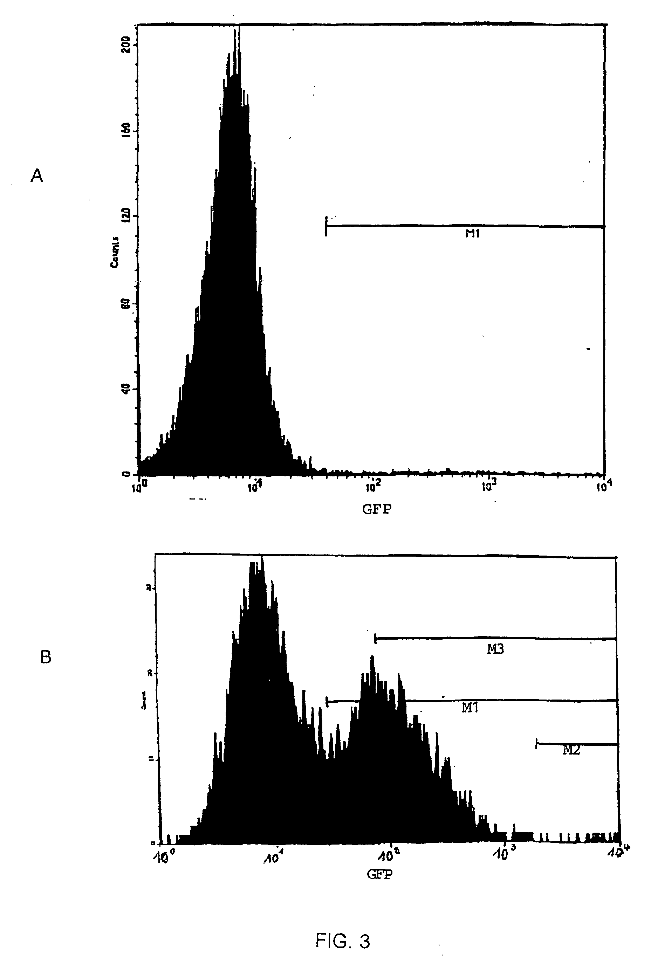

Die FACS-Analyse bewies eine hohe Effektivität der Puromycin-Selektion der verwendeten transgenen ES-Zellen. Während die EGFP-fluoreszierenden Zellen nur ungefähr 1% Anteil der Gesamtzellpopulation unbehandelter Zellen darstellten, die einen pαMHC-EGFP-Vektor besaßen, führte die Puromycin-Behandlung von differenzierenden embryonale Stammzellen, die sowohl bezüglich pαMHC-EGFP als auch pαMHC-Pur Vektoren transgen waren, zu einer 42 - 45%igen Anreicherung der Zellpopulation durch EGFP-fluoreszierende Zellen (Fig. 3). Die einfache Berechnung zeigt, daß bereits 97 - 99% der gesamten nicht kardiogenen Zellpopulation effektiv im Verlauf der Puromycin-Behandlung der Suspensionskultur von transgenen ES-Zellen getötet wurden. Die noch existierende Fraktion an Puromycin-resistenten nicht- oder -schwach fluoreszierenden Zellen (Fig. 3) konnte durch eine nicht-spezifische Aktivität des αMHC-Promotors in einigen der nicht kardiogenen Zellen erklärt werden. Eine derartige Fraktion konnte weiter entweder durch Höchstkonzentrationen von Puromycin oder durch FACS -Sortierverfahren eliminiert werden.

### Beispiel 2

Als Ausgangsvektor wurde pIRES2-EGFP (Clontech Laboratories, Palo Alto, CA) eingesetzt. Dieser Vektor enthält die interne Ribosomeneintrittstelle (IRES) des Encephalo-Myocarditis-Virus zwischen der multiplen Klonierungsstelle (MCS) und dem EGFP-Gen. Dies ermöglicht, daß sowohl die Puromycinresistenzals auch die EGFP-Gene getrennt von einer einzigen bicistronischen mRNA translatiert werden.

Der pIRES2-EGFP-Vektor wurde mit den Restriktionsenzymen AseI und Eco47III an den Enden stumpf gemacht und religiert, um den Cytomegalo-Virus immediate early (CMV-IE)-Promotor zu deletieren. Der so erhaltene Vektor wurde mit SmaI verdaut und mit der α-MHC-PurR-Kassette ligiert, die durch SacI und ClaI aus dem oben beschriebenen α-MHC-PurR-Vektor herausgeschnitten worden war. Durch Verdauen mit SacI/SmaI wurde die richtige Orientierung des so erhaltenen pα-MHC-IHRES-EGFP (pα-PIG)-Vektors sichergestellt.

ES-Zellen (D3-Zelllinie) wurden mit pα-PIG transfiziert; die nachfolgende G418-Selektion, die Vermehrung und die Differenzierung der so erhaltenen stabilen Klone wurde, wie bereits im Beispiel 1 beschrieben, durchgeführt.

Nach Durchführung des Standard-Differenzierungsprotokolls konnte man schlagende Cluster EGFP-positiver Herzzellen zwischen dem achten und neunten Tag der Entwicklung nachweisen, worauf Puromycin 5µg/ml zugegeben wurde. Nach den ersten drei bis vier Tagen der Puromycinbehandlung enthielten die embryoiden Körperchen (EBs) hauptsächlich EGFP-positive, intensiv schlagende Cluster an Herzzellen; nicht-Herzzellen lösten sich ab und wurden während des Mediumwechsels eliminiert. Das gleiche Ergebnis konnte auch dadurch erzielt werden, daß man die EBs vollständig in Suspensionskultur wachsen ließ und dort die Resistenzbehandlung mit dem Antibiotikum durchführte. Eine FACS-Analyse zeigte eine Anreicherung von zumindest 70% (Durchflußzytometrie unter Verwendung von EGFP als read out) in der so erhaltenen Zellkultur. Die Anordnung des Reportergens und des Resistenzgens auf einem Vektor unter Kontrolle eines Promotors, bevorzugt in Verbindung mit einer I-RES, eignet sich somit hervorragend zur Herstellung differenzierter embryonaler Stammzellen, die so weitgehend wie möglich frei sind von undifferenzierten Stammzellen. Das gleiche gilt natürlich für Keimbahnzellen bzw. adulte Stammzellen und nicht nur für embryonale Stammzellen. Insbesondere konnte durch dieses Beispiel gezeigt werden, daß eine außerordentlich hohe Gewebespezifität für sich aus ES-Zellen entwickelnde Herzzellen erreichbar ist.

### Gültigkeit des Puromycin-Selektionsprotokolls

Das Puromycin-Selektionsverfahren wurde anschließend in einem autologen Maus-Modell, in welchem eine Verletzung des Herzens simuliert wurde, getestet und konnte so validiert werden. Zu diesem Zwecke wurde ein Maus-Transplantations-Modell eingesetzt, in welchem embryonale Stammzellen oder durch in vitro-Differenzierung von ES-Zellen erhaltene Herzzellen (10.000 - 100.000 Zellen) in einen Empfänger injiziert wurden, dessen Herz durch eine Kältebehandlung teilgeschädigt war. Die Entstehung von Tumoren wurde anhand der gesamten Maus, dem isolierten Herzen und auch an Gewebeschnitten morphologisch untersucht; diese Untersuchungen wurden zu verschiedenen Zeiten nach der Operation im Zeitraum von zwei Tagen bis zwei Monaten durchgeführt. Diese Vorgehensweise ermöglichte eine genaue Bewertung des Tumorpotentials der verschiedenen Zellpräparationen. Bei Injektion nicht-differenzierter ES-Zellen in die Kryo-Verletzung (100.000 Zellen) entstanden große Tumoren in den Mäusen. 10 Tage nach der Operation erlagen die Tiere diesen Tumoren. Es entstanden aber auch dann Tumoren, wenn ES-Zellen in vitro zu Herzzellen differenzierten und die schlagenden Bereiche, die für von ES-Zellen abstammenden Kardiomyozyten typisch sind, abgetrennt, isoliert und 10.000 bis 50.000 Zellen hiervon in die Mäuse injiziert wurden. Dies beweist das hohe Tumorpotential embryonaler Stammzellen im Herzen und die hohen Anforderungen, die an ein hochspezifisches Selektionsverfahren zu stellen sind.

In einem nächsten Versuch wurden transgene ES-Zellen, die mit dem erfindungsgemäßen Konstrukt stabil transfiziert waren (Reportergen und Resistenzgen unter Kontrolle eines Promortors auf einem Vektor), nach dem Nachweis der EGFP-Expression für fünf bis sieben Tage einer Puromycinbehandlung unterzogen. In einer groß angelegten Versuchsreihe von mehr als 25 operierten Mäusen, die alle am Herzen der oben beschriebenen Kryo-Behandlung unterzogen worden waren, konnte selbst nach mehreren Monaten keine Bildung von Tumoren beobachtet werden, wenn diese von puromycinresistenten ES-Zellen abstammenden Zellen (10.000 - 50.00-0 Zellen) in die verletzten Maus-Herzareale injiziert wurden (Doppeltransfektions-Konstrukte). Tatsächlich gelang es, die Zellen nach der Transplantation zu identifizieren, und es konnte deutlich gezeigt werden, daß die Zellen erfolgreich transplantiert werden konnten und sich zu terminal differenzierten Kardiomyozyten weiter differenzierten. Diese Versuche beweisen damit eindeutig die Fähigkeit der erfindungsgemäß beschriebenen Technik, von ES-Zellen abstammende, in vitro-differenzierte Zellen effizient anzureichern und eine Population zu erhalten, in der sich keine undifferenzierten ES-Zellen mehr befinden. Diese Effizienz ist unter Berücksichtigung der hier gezeigten hohen Tumorogenität von von ES-Zellen abstammenden Zellen im Herzen besonders bemerkenswert.

### SCHLUßFOLGERUNGEN

1. Stabile transgene embryonalen Stammzell-Klone, die mit den Expressions-Vektoren pαMHC-EGFP und pαMHC-Pur kotransfiziert waren, wurden gebildet.
2. Eine Puromycin-Behandlung der transgenen embryonalen Stammzellen im Verlaufe der Differenzierung in vitro zeigte eine hohe Effizienz der kardiospezifischen Selektion im Vergleich mit einer Hygromycin-Behandlung von früher erzeugten pαMHC-Hyg ES-Zelllinien (Daten nicht angegeben).
3. Die selektierten differenzierten Zellen zeigten einen höheren Grad an morphologischer und funktioneller Lebensfähigkeit und Langlebigkeit als ihre unbehandelten Gegenstücke, was nahelegt, daß der genetische Selektions-Ansatz differenzierende embryonale Stammzellen effektiv von negativen Einflüssen der sie umgebenden Zellen befreit.
4. Die kombinierte Anwendung von *live*-Fluoreszenz-Reporterund Arzneistoff-Resistenzgenen unter einem gemeinsamen Zelltyp-spezifischen Promotor haben die gestraffte Überwachung und Quantifizierung eines Gesamtprozesses ermöglicht, einschließlich der Differenzierung und der Zelltyp-spezifischen Selektion. Die sich ergebenden Zellen sind für weitere Transplantations-Experimente einsetzbar, was das Überwachen eingefügter Zellen ermöglicht.
4. Der dargelegte Ansatz kann auf irgendeine Zelltypspezifische Selektion in einem ES-Zell-Differenzierungssystem angewendet werden, wenn ein hochspezifischer Promotor für einen entsprechenden Zelltyp oder ein spezifisches Entwicklungsstadium identifiziert und kloniert ist. Prinzipiell erlaubt das System die kombinierte Verwendung zweier unterschiedlicher Promotoren mit entsprechenden zweifarbigen in vivo fluoreszierenden Proteinen, beispielsweise den Gelb (EYFP) und Cyan (blau) (ECFP) Versionen von EGFP, und zwei Wirkstoff-Resistenzgenen. Ein derartiger Ansatz kann die Selektivität und Effizienz des Gesamtverfahrens weiter erhöhen.

Die erfindungsgemäß bereitgestellten embryonalen Stammzellen, bevorzugt embryoide Körperchen, können zu toxikologischen Untersuchungen von Substanzen, beispielsweise Schwermetallen und Pharmaka, eingesetzt werden (siehe auch Auflistung weiter oben). Zu diesem Zweck werden embryonale Stammzellkulturen unter Verwendung des doppelten Vektorkonstrukts verwendet und das Selektionsagens nach Beginn der zelltypischen Differenzierung (Detektion der Fluoreszenz) hinzugegeben. Nach der Zellaufreinigung oder aber bereits während der ES-Zellkultivierung werden die unterschiedlichen zu testenden Substanzen zur Zellkultur hinzugegeben und zu verschiedenen Zeiten die fluoreszierenden Einzelzellen bzw. die Gesamtfluoreszenz mit unterschiedlichen Auslesemethoden (z.B. Durchflusszytometrie, Fluoreszenzreadern) im Vergleich zu den Kontrollen gemessen.

Die erfindungsgemäß bereitgestellten embryonalen Stammzellen können zur Erzeugung transgener nicht menschlicher Säuger mit zellspezifischer oder entwicklungsspezifischer Expression des fluoreszierenden Proteins eingesetzt werden. Hierbei werden die beschriebenen ES-Zellen der Erfindung in Blastozysten von nicht menschlichen Säugern eingebracht. In einem nächsten Schritt werden die Blastozysten als Chimären, die durch Kreuzung homozygot werden, in Leihmütter übertragen und so transgene nicht menschliche Säuger erhalten.

In einer weiteren Ausgestaltungsform der Erfindung werden die transgenen embryonalen Stammzellen in Form einer pharmazeutischen Zusammensetzung zu Transplantationszwecken eingesetzt. Zu diesem Zweck werden hochaufgereinigte embryonale Stammzell-abgeleitete Kulturen benötigt, da bekannt ist, daß eine Kontamination mit undifferenzierten proliferierenden Stammzellen zur Tumorbildung führt. Dementsprechend eignet sich das hier beschriebene Verfahren ideal, hochaufgereinigte ES Zell-abgeleitete zellspezifische Kulturen zu erhalten, die sich ideal zur Transplantation eignen (Klug et al., 1996).

Es sei abschließend noch einmal betont, daß die vorstehend an Hand embryonaler Stammzellen dargestellte Erfindung auch auf embryonale Keimbahnzellen, und auf adulte Stammzellen übertragbar ist.

Die vorliegende Erfindung offenbart ein System zur zell- und entwicklungs-spezifischen Selektion von differenzierenden embryonalen oder adulter Stammzellen oder embryonaler Keimbahnzellen durch kombinierte Anwendung von Resistenz- und detektierbaren Reportergenen unter gemeinsamer Kontrolle eines zell- und/oder entwicklungs-spezifischen Promotors.

Vom Schutzbereich sind jedoch menschliche embryonale Stamm- und Keimzellen, sowie deren Verwendung im erfinderischen Verfahren, ausdrucklich ausgeschlossen.

### Literatur

Anderson, D.J., Gage, F.H., and Weissmann, I.L. (2001). Can stem cells cross lineage boundaries? Nat. Med. 7, 393-395.
Doetschman,T.C., Eistetter,H., Katz,M., Schmidt,W., and Kemler,R. (1985). The in vitro development of blastocyst-derived embryonic stem cell lines: formation of visceral yolk sac, blood islands and myocardium. J. Embryol. Exp. Morphol. 87, 27-45.
Gage, F.H. (2000). Mammalian neural stem cells. Science 287, 1433-1438.
Heim,R. and Tsien,R.Y. (1996). Engineering green fluorescent protein for improved brightness, longer wavelengths and fluorescence resonance energy transfer. Curr. Biol. 6, 178-182.
Kallmeier,R.C., Somasundaram,C., and Babij,P. (1995). A novel smooth muscle-specific enhancer regulates transcription of the smooth muscle myosin heavy chain gene in vascular smooth muscle cells. J. Biol. Chem. 270, 30949-30957.
Klug,M.G., Soonpaa,M.H., Koh,G.Y., and Field,L.J. (1996). Genetically selected cardiomyocytes from differentiating embronic stem cells form stable intracardiac grafts. J Clin. Invest. 98, 216-224.
Kolossov,E., Fleischmann,B.K., Liu,Q., Bloch,W., Viatchenko-Karpinski,S., Manzke,O., Ji,G.J., Bohlen,H., Addicks,K., and Hescheler,J. (1998). Functional characteristics of ES cellderived cardiac precursor cells identified by tissue-specific expression of the green fluorescent protein. J Cell Biol 143, 2045-2056.
Lints,T.J., Parsons,L.M., Hartley,L., Lyons,I., and Harvey,R.P. (1993). Nkx-2.5: a novel murine homeobox gene expressed in early heart progenitor cells and their myogenic descendants [published erratum appears in Development 1993 Nov;119(3):969]. Development 119, 419-431.
Lothian,C. and Lendahl,U. (1997). An evolutionarily conserved region in the second intron of the human nestin gene directs gene expression to CNS progenitor cells and to early neural crest cells. Eur J Neurosci. 9, 452-462.
Muldoon,R.R., Levy,J.P., Kain,S.R., Kitts,P.A., and Link,C.J., Jr. (1997). Tracking and quantitation of retroviral-mediated transfer using a completely humanized, redshifted green fluorescent protein gene. Biotechniques 22, 162-167.
Muller M, Fleischmann B.K., Selbert S, Ji G.J., Endl E, Middeler G, Mueller OJ, Schlenke P, Frese S, Wobus AM, Hescheler J, Katus, H. A., and Franz, W. M. Selection of ventricularlike cardiomyocytes from ES cells in vitro. FASEB J . 2000. Ref Type: In Press
O'Brien,T.X., Lee,K.J., and Chien,K.R. (1993). Positional specification of ventricular myosin light chain 2 expression in the primitive murine heart tube. Proc. Natl. Acad. Sci. U. S. A. 90, 5157-5161.
Prockop, D.J. (1997). Marrow stromal cells as stem cells for nonhematopoietic tissues. Science 276, 71-74.
Sartorelli,V., Webster,K.A., and Kedes,L. (1990). Muscle-specific expression of the cardiac alpha-actin gene requires MyoD1, CArG-box binding factor, and Sp1. Genes Dev. 4, 1811-1822.
Shamblott MJ, Axelmann J, Wang S, Bugg EM, Littlefield JW, Donovan PJ, Blumenthal PD, Huggins GR, and Gearhart JD (1998). Derivation of pluripotent stem cells from cultured human primordial germ cells. Proc. Natl. Acad. Sci. U. S. A. 95, 13726-13731.
Thomson JA, Itskovitz-Eldor J, Shapiro SS, Waknitz MA, Swiergiel JJ, Marshall VS, and Jones JM (1998). Embryonic stem cell lines derived from human blastocysts. Science 282, 1145-1147.
Wartenberg,M., Donmez,F., Ling,F.C., Acker,H., Hescheler,J., and Sauer,H. (2001). Tumor-induced angiogenesis studied in confrontation cultures of multicellular tumor spheroids and embryoid bodies grown from pluripotent embryonic stem cells. FASEB J 2001. Apr;15. (6. ):995. -1005. 15, 995-1005.
Wobus,A.M., Wallukat,G., and Hescheler,J. (1991). Pluripotent mouse embryonic stem cells are able to differentiate into cardiomyocytes expressing chronotropic responses to adrenergic and cholinergic agents and Ca2+ channel blockers. Differentiation. 48, 173-182.

## Patentansprüche

1. Verfahren zur Gewinnung von Herzzellen oder von sich zu Herzzellen differenzierenden Stammzellen, außer menschlichen umfassend:
(a) das Einführen zumindest eines Vektors enthaltend DNA-Sequenzen mit der Information für zumindest ein Reportergen, das ein nicht zellschädigendes fluoreszierendes Protein kodiert, und zumindest ein Resistenzgen das für eine Resistenz gegen Puromycin verantwortlich ist, wobei die DNA-Sequenzen beide unter der Kontrolle desselben Promotors stehen, der aus einem herzzellspezifischen Promotor ausgewählt ist und operabel mit den Genen verbunden ist in Stammzellen außer menschlicher embryonaler Stammzellen; wobei die Zellenstabil transfiziert werden, und
(b) Nachweis der Expression des Reportergens;
(c) Zugabe eines Selektionsmittels zur Selektion auf die das Reportergen und das Resistenzgen exprimierenden Zellen;
(d) Gewinnen der aus den Stammzellen unter Steuerung des herazell-spezifischen Promotors sich entwickelnde sich differenzierenden oder differenzierten Zellen.

2. Verfahren nach Anspruch 1, wobei die Zellen Säugetierzellen sind.

3. Verfahren nach Anspruch 2, wobei die Säugetierzellen von Primaten oder Nagetieren, insbesondere von Mäusen, Ratten oder Kaninchen, stammen, oder menschlichen Ursprungs sind.

4. Verfahren nach einem oder mehreren der vorhergehenden Ansprüche, bei denen das nicht zellschädigende fluoreszierende Protein ausgewählt ist aus (enhanced) Grün Fluoreszierendem Protein (EGFP und GFP), Rot Fluoreszierendem Protein (RFP), Blau Fluoreszierendem Protein (BFP), Gelb Fluoreszierendem Protein (YFP) und Cyan Fluoreszierendem Protein (CFP), insbesondere GFP.

5. Verfahren nach einem oder mehreren der vorhergehenden Ansprüche, bei denen der herzspezifische Promotor aus Nkx-2.5-, menschlichem α-Aktin-, α-MHC-, β-MHC, ANF-, Brachyuria und MLC-2V-Promotoren ausgewählt ist.

6. Verfahren nach einem oder mehreren der vorhergehenden Ansprüche, bei denen der Promotor mit weiteren funktionellen DNA-Sequenzen, insbesondere Enhancer-Sequenzen oder Repressor-Sequenzen oder IRES-Sequenzen, verbunden ist.

7. Verfahren nach einem oder mehreren der vorhergehenden Ansprüche, bei denen der herzzell-spezifische Promotor mit einem Puromycin-Resistenzgen operabel verbunden ist.

8. Verfahren nach einem oder mehreren der vorhergehenden Ansprüche, wobei
(a) die Zellen stabil zwei Vektoren enthalten, von denen der erste Vektor DNA-Sequenzen enthält, die für zumindest ein Reportergen kodieren, und der zweite Vektor DNA-Sequenzen enthält, die für zumindest ein Resistenzgen kodieren, wobei die DNA-Sequenzen beide unter der Kontrolle desselben Promotors stehen; oder
(b) die Zellen einen Vektor enthalten, der DNA-Sequenzen enthält, die für zumindest ein Reportergen und zumindest ein Resistenzgen kodieren, die beide auf ein- und demselben Vektor liegen und unter Kontrolle ein und desselben Promotors stehen, wobei bevorzugt eine TRES-Sequenz, bevorzugt zwischen dem Reportergen und dem Resistenzgen, angeordnet ist, oder
(c) die Zelle zwei Sätze selektiver Vektor-Systeme enthält, umfassend
- einen ersten Vektor mit DNA-Sequenzen, die für ein Reportergen und ein erstes Resistenzgen kodieren, die beide unter der Kontrolle eines ersten zell- und/oder entwicklungs-spezifischen Promotors stehen, der operabel mit diesen Sequenzen verbunden ist;
- einen zweiten Vektor, der DNA-Sequenzen umfasst, die für ein zweites Reportergen und ein zweites Resistenzgen kodieren, die beide unter Kontrolle eines zweiten zell- und/oder entwicklungsspezifischen Promotors stehen, der operabel mit diesen Sequenzen verbunden ist.

9. Verfahren nach einem oder mehreren der vorhergehenden Ansprüche, wobei die Zellen ein weiteres Resistenzgen zur Selektion auf die mit den Vektor-Konstrukten stabil transfizierten Zellen enthalten, welches verschieden ist von dem ersten und zweiten Resistenzgen.

10. Verfahren nach einem oder mehreren der vorhergehenden Ansprüche, wobei die Zellen als Zellaggregate, insbesondere in Form von embryoiden Körperchen kultiviert werden.

11. Verfahren nach einem der Ansprüche 1 bis 10 bei dem der Vektor oder die Vektoren durch Transfektion, Elektroporation, Virusvektoren oder Lipofektion eingeführt wird.

12. Verfahren nach einem der Ansprüche 1 bis 11, wobei das Selektionieren auf die den Vektor enthaltenden Zellen vor Schritt (a) die Zugabe eines ersten Selektionsmittels zur Selektion von stabil transfizierten Zellen umfasst.

13. Verfahren nach einem oder mehreren der vorhergehenden Ansprüche, wobei die pluripotenten Stammzellen in Form von embryoiden Körperchen oder in Kokultur mit anderen Zellen kultiviert werden.

14. Verfahren nach einem oder mehreren der vorhergehenden Ansprüche, wobei die Zellen in einer Suspension kultiviert werden und/oder die Zellen als "embryoid bodies" kultiviert werden.

15. Verfahren nach einem oder mehreren der vorhergehenden Ansprüche, wobei Puromycin als Selektionsmittel zur Selektion der das Reportergen exprimierenden Zellen eingesetzt wird und/oder wobei die selektierten Zellen einer Zellsortierung zur weiteren Anreicherung unterzogen werden.

16. Zellkultur, außer eine Zellkultur menschlicher embryoalen Stammzellen, die eine zell- oder entwicklungs-spezifische Expression eines Reportergens und eines Resistenzgens zeigt, wobei die Zellen das Vektor-Konstrukt bzw. die Vektor-Konstrukte wie in einem oder mehreren der vorhergehenden Ansprüche definiert enthalten.

17. Herzzellen oder sich zu Herzzellen differenzierende Stammzellen außer menschliche embryonale Stammzellen, die das Vektor-Konstrukt bzw. die Vektor-Konstrukte wie in einem der Ansprüche 1 bis 9 definiert enthalten.

18. Verfahren zur toxikologischen Überprüfung von Substanzen mit den nachfolgende Schritten:
- Bereitstellen einer Zellkultur nach Anspruch 16 oder Zellen nach Anspruch 17;
- Einbringen von Substanzen, deren toxische oder nichttoxische Wirkungen getestet werden sollen, in die Zellkultur;
- quantitative und/oder qualitative Bestimmung der Fluoreszenz der so erhaltenen Zellen und Vergleich mit Zellen, die ohne die zu untersuchende Substanz kultiviert wurden.

## Claims

1. A method for obtaining cardiac cells or stem cells, except human embryonic stem cells, differentiating into cardiac cells, said method comprising:
(a) introducing into stem cells, except human embryonic stem cells, at least one vector containing DNA sequences containing the information for at least one reporter gene coding for a non-cell damaging fluorescent protein and at least one resistance gene that is responsible for a resistance to puromycin, wherein both DNA sequences are under the control of the same promoter which is selected from a cardiac cell-specific promoter and is operably linked to said genes, wherein said cells are stably transfected; and
(b) detecting the expression of said reporter gene;
c) adding a selective agent for selection for those cells expressing the reporter gene and the resistance gene;
d) obtaining the differentiated or differentiating cells developing from said stem cells under the control of the cardiac cell-specific promoter.

2. The method according to claim 1, wherein said cells are mammalian cells.

3. The method according to claim 2, wherein the mammalian cells are derived from primates or rodents, particularly from mice, rats or rabbits or are of human origin.

4. The method according to one or more of the preceding claims, wherein said non-cell damaging fluorescent protein is selected from (enhanced) Green Fluorescent Protein (EGFP and GFP), Red Fluorescent Protein (RFP), Blue Fluorescent Protein (BFP), Yellow Fluorescent Protein (YFP) and Cyan Fluorescent Protein (CFP), in particular GFP.

5. The method according to one or more of the preceding claims, wherein the cardiac cell-specific promoter is selected from Nkx-2.5, human α actin, α MHC, β MHC, ANF, Brachyury and MLC-2V promoters.

6. The method according to one or more of the preceding claims, wherein the promoter is linked to further functional DNA sequences, in particular enhancer sequences or repressor sequences or IRES sequences.

7. The method according to one or more of the preceding claims, wherein the cardiac cell-specific promoter is operably linked to a puromycin resistance gene.

8. The method according to one or more of the preceding claims, wherein
a) the cells stably contain two vectors, wherein the first vector contains DNA sequences coding for at least one reporter gene and the second vector contains DNA sequences coding for at least one resistance gene, wherein both DNA sequences are under the control of the same promoter; or
b) the cells contain one vector containing DNA sequences coding for at least one reporter gene and at least one resistance gene, both located on one and the same vector and being under the control of one and the same promoter, wherein preferably an IRES sequence is preferably located between the reporter gene and the resistance gene; or
c) the cell contains two sets of selective vector systems, comprising
- a first vector having DNA sequences coding for a first reporter gene and a first resistance gene, both being under the control of a first cell- and/or development-specific promoter that is operably linked to said sequences;
- a second vector having DNA sequences coding for a second reporter gene and a second resistance gene, both being under the control of a second cell- and/or development-specific promoter that is operably linked to said sequences.

9. The method according to one or more of the preceding claims, wherein said cells contain a further resistance gene for selection for those cells being stably transfected with the vector constructs, which resistance gene is different from the first and second resistance gene.

10. The method according to one or more of the preceding claims, wherein said cells are cultivated as cell aggregates, in particular in form of embryoid bodies.

11. The method according to any one of claims 1 to 10, wherein the vector/s is/are introduced by means of transfection, electroporation, virus vectors or lipofection.

12. The method according to any one of claims 1 to 11, wherein the selection for those cells containing the vector comprises, prior to step (a), adding a first selective agent for selecting stably transfected cells.

13. The method according to one or more of the preceding claims, wherein said pluripotent stem cells are cultivated in form of embryoid bodies or in co-culture with other cells.

14. The method according to one or more of the preceding claims, wherein said cells are cultivated in a suspension and/or as "embryoid bodies".

15. The method according to one or more of the preceding claims, wherein puromycin is employed as selective agent for selecting those cells expressing the reporter gene and/or wherein the selected cells are subjected to cell sorting for further enrichment.

16. A cell culture, except a cell culture of human embryonic stem cells, exhibiting a cell- and/or development-specific expression of a reporter gene and a resistance gene, wherein said cells contain the vector construct/s as defined in one or more of the preceding claims.

17. Cardiac cells or stem cells differentiating into cardiac cells, except human embryonic stem cells, containing the vector construct/s as defined in any one of claims 1 to 9.

18. A method for toxicological testing of substances, said method comprising the following steps:
- providing a cell culture according to claim 16 or cells according to claim 17;
- introducing substances, whose toxic or non-toxic effects are to be tested, into said cell culture;
- quantitative and/or qualitative testing of the fluorescence of the cells thus obtained and comparing said cells to cells that were cultured without the substance to be tested.

## Revendications

1. Procédé d'obtention de cellules cardiaques ou de cellules souches se différenciant en cellules cardiaques, hormis de cellules souches embryonnaires humaines, comprenant :
(a) l'introduction d'au moins un vecteur contenant des séquences d'ADN portant l'information pour au moins un gène reporter qui code une protéine fluorescente non cytotoxique et au moins un gène de résistance qui est responsable d'une résistance à la puromycine, les deux séquences d'ADN étant sous le contrôle du même promoteur qui est choisi parmi des promoteurs spécifiques des cellules cardiaques et lié de manière fonctionnelle aux gènes, dans des cellules souches, hormis des cellules souches embryonnaires humaines, les cellules étant transfectées de manière stable ; et
(b) mise en évidence de l'expression du gène reporter ;
(c) ajout d'un moyen de sélection pour la sélection sur les cellules exprimant le gène reporter et le gène de résistance ;
(d) obtention des cellules différenciées ou en voie de différenciation se développant à partir des cellules souches sous le contrôle du promoteur spécifique des cellules cardiaques.

2. Procédé selon la revendication 1, dans lequel les cellules sont des cellules de mammifères.

3. Procédé selon la revendication 2, dans lequel les cellules de mammifères proviennent de primates ou de rongeurs, en particulier de souris, rats ou lapins, ou sont d'origine humaine.

4. Procédé selon une ou plusieurs des revendications précédentes, dans lequel la protéine fluorescente non cytotoxique est choisie parmi une protéine fluorescente verte (améliorée) (EGFP et GFP), une protéine fluorescente rouge (RFP), une protéine fluorescente bleue (BFP), une protéine fluorescente jaune (YFP) et une protéine fluorescente cyan (CFP), en particulier une GFP.

5. Procédé selon une ou plusieurs des revendications précédentes, dans lequel le promoteur spécifique cardiaque est choisi parmi des promoteurs de Nkx2.5, de l'α-actine humaine, de l'α-MHC, de la β-MHC, de l'ANF, de Brachyury et de la MLC-2V.

6. Procédé selon une ou plusieurs des revendications précédentes, dans lequel le promoteur est lié à d'autres séquences d'ADN fonctionnelles, en particulier des séquences activatrices, des séquences inhibitrices ou des séquences IRES.

7. Procédé selon une ou plusieurs des revendications précédentes, dans lequel le promoteur spécifique de cellules cardiaques est lié de manière fonctionnelle à un gène de résistance à la puromycine.

8. Procédé selon une ou plusieurs des revendications précédentes, dans lequel
(a) les cellules contiennent de manière stable deux vecteurs dont le premier vecteur contient des séquences d'ADN qui codent au moins un gène reporter et le deuxième vecteur contient des séquences d'ADN qui codent au moins un gène de résistance, les deux séquences d'ADN étant sous le contrôle du même promoteur ; ou
(b) Les cellules contiennent un vecteur qui contient des séquences d'ADN qui codent au moins un gène reporter et au moins un gène de résistance qui sont tous deux situés sur un seul et même vecteur et sont sous le contrôle d'un seul et même promoteur, de préférence une séquence IRES étant disposée de préférence entre le gène reporter et le gène de résistance, ou
(c) la cellule contient deux jeux de systèmes de vecteur sélectifs, comprenant
- un premier vecteur avec des séquences d'ADN qui codent un gène reporter et un premier gène de résistance qui sont tous deux sous le contrôle d'un premier promoteur spécifique des cellules et/ou du développement, qui est lié de manière fonctionnelle à ces séquences ;
- un deuxième vecteur avec des séquences d'ADN qui codent un deuxième gène reporter et un deuxième gène de résistance qui sont tous deux sous le contrôle d'un deuxième promoteur spécifique des cellules et/ou du développement, qui est lié de manière fonctionnelle à ces séquences.

9. Procédé selon une ou plusieurs des revendications précédentes, dans lequel les cellules contiennent un autre gène de résistance pour la sélection sur les cellules transfectées de manière stable avec les constructions de vecteurs, lequel est différent du premier et du deuxième gène de résistance.

10. Procédé selon une ou plusieurs des revendications précédentes, dans lequel les cellules sont cultivées sous la forme d'agrégats de cellules, en particulier sous la forme de corps embryoïdes.

11. Procédé selon une des revendications 1 à 10, dans lequel le vecteur ou les vecteurs sont introduits par transfection, électroporation, vecteurs viraux ou lipofection.

12. Procédé selon une des revendications 1 à 11, dans lequel la sélection sur les cellules contenant le vecteur comprend avant l'étape (a) l'ajout d'un premier moyen de sélection pour la sélection de cellules transfectées de manière stable.

13. Procédé selon une ou plusieurs des revendications précédentes, dans lequel les cellules souches pluripotentes sont cultivées sous la forme de corps embryoïdes ou en coculture avec d'autres cellules.

14. Procédé selon une ou plusieurs des revendications précédentes, dans lequel les cellules sont cultivées dans une suspension et/ou les cellules sont cultivées sous la forme de corps embryoïdes (« embryoid bodies »).

15. Procédé selon une ou plusieurs des revendications précédentes, dans lequel de la puromycine est utilisée comme moyen de sélection pour la sélection des cellules exprimant le gène reporter et/ou dans lequel les cellules sélectionnées sont soumises à un tri cellulaire pour un enrichissement additionnel.

16. Culture cellulaire, hormis une culture cellulaire de cellules souches embryonnaires humaines, qui montre une expression spécifique des cellules ou du développement d'un gène reporter et d'un gène de résistance, les cellules contenant la construction de vecteur ou les constructions de vecteurs comme défini dans une ou plusieurs des revendications précédentes.

17. Cellules cardiaques ou cellules souches se différenciant en cellules cardiaques, hormis cellules souches embryonnaires humaines, qui contiennent la construction de vecteur ou les constructions de vecteurs comme défini dans une des revendications 1 à 9.

18. Procédé d'analyse toxicologique de substances, comprenant les étapes suivantes :
- mise à disposition d'une culture cellulaire selon la revendication 16 ou de cellules selon la revendication 17 ;
- introduction dans la culture cellulaire de substances dont les effets toxiques ou non toxiques doivent être contrôlés ;
- détermination quantitative et/ou qualitative de la fluorescence des cellules ainsi obtenues et comparaison avec des cellules qui ont été cultivées sans la substance à analyser.
